# EUROPEAN PATENT APPLICATION

(11) **EP 4 324 829 A1**
(43) Date of publication of application: **21.02.2024**
(21) Application number: 22788374.1
(22) Date of filing: 11.04.2022
(51) Int. Cl.: C07D 405/12, A61K 31/541, C12N 5/077

(54) **SULFATE OF 5-[(1,1-DIOXIDO-4-THIOMORPHOLINYL)METHYL]-2-PHENYL-N-(TETRAHYDRO-2H-PYRAN-4-YL)-1H-INDOLE-7-AMINE, AND NOVEL CRYSTAL FORM THEREOF**

(30) Priority: 12.04.2021 KR 20210047170
(71) Applicant: MitoImmune Therapeutics Inc., Seoul 06123 (KR)
(72) Inventor: KIM, Soon Ha, Seoul 06123 (KR); LEE, Sae Yeon, Seoul 06123 (KR); KIM, Hyoung Jin, Seoul 06123 (KR); CHANG, Hye Kyung, Seoul 06123 (KR); JEON, Sang Kweon, Seoul 06123 (KR); SEO, Mooyoung, Seoul 06123 (KR)
(74) Representative: Plasseraud IP
(86) International application number: PCT/KR2022/005210
(87) International publication number: WO 2022/220518

(57) **Abstract**

The present invention relates to a sulfate of a compound of chemical formula 1, and a crystalline form thereof. The sulfate of the compound of chemical formula 1, according to the present invention, has much higher solubility than other salts thereof, and exhibits excellent stability and absorption into the body. In addition, such a sulfate can form various crystalline forms, and these crystalline forms are also stable and undergo almost no reduction in purity, even after long-term storage under various conditions, and thus are useful for storage.

## Description

### [Technical Field]

The present invention relates to a sulfate of a compound of the following Chemical Formula 1, 5-[(1,1-dioxido-4-thiomorpholinyl)methyl]-2-phenyl-N-(tetrahydro-2H-pyran-4-yl)-1H-indol-7-amine, and a crystalline form thereof.

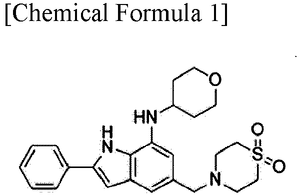

### [Background Art]

Various salts or crystalline forms of the same drug are able to exhibit various solid state physical properties, such as hygroscopicity, compressive behavior, stability during storage and flowability of milled solids. These properties affect their suitability as an active pharmaceutical substance for commercial production in a particular solid state. For example, flowability affects the ease of handling during processing of a substance into a pharmaceutical product. When powdered compound particles do not flow easily, formulation specialists will have to consider that fact in the development of formulations such as tablets or capsules, and it may be necessary to use a lubricant such as colloidal silicon dioxide, talc, starch or tribasic calcium phosphate.

In addition, various salts or crystalline forms of the same drug may have substantial differences in pharmaceutically important properties such as dissolution rate and bioavailability. The dissolution rate is not only considered in the preparation of syrups, elixirs and other liquid medicaments but may also change treatment outcomes. For example, the dissolution rate of an active ingredient in a patient's gastric fluid causes varying treatment outcomes because it places an upper limit on the rate at which an orally administered active ingredient can reach the patient's bloodstream.

Meanwhile, the compound of Chemical Formula 1, 5-[(1,1-dioxido-4-thiomorpholinyl)methyl]-2-phenyl-N-(tetrahydro-2H-pyran-4-yl)-1H-indol-7-amine, is a compound disclosed in International Patent Publication No. WO2009-025478 and is known to exhibit an effect of preventing or treating and ameliorating necrosis and necrosis-related diseases. However, the compound of Chemical Formula 1 has very low solubility in not only water but also many other solvents.

Very low solubility in various solvents adversely affects pharmaceutical development that requires the production of a finished product in a completely dissolved state. As an example, in the development of an injection, when a main ingredient and an excipient are in a completely dissolved state, an accurate dosage of a finished product formulation is achieved. As another example, in the case of a finished product for oral use, the most commonly used spray drying method also requires complete dissolution of an active pharmaceutical ingredient, and this requirement can lead to the production of a finished product with a uniform dosage during the process. The solubility of a main ingredient also has a great influence on the development of topical formulations such as creams, gels or ointments, patches and inhalation formulations.

Accordingly, the present inventors have continuously conducted research on the salt form of the compound of Chemical Formula 1 for improving the solubility of the compound of Chemical Formula 1. As a result, the present inventors have found the best salt of the compound of Chemical Formula 1 in terms of a physicochemical aspect and identified various crystalline forms of the salt and characteristics thereof.

### [Related-Art Documents]

### [Patent Documents]

International Patent Publication No. WO2009-025478

### [Disclosure]

### [Technical Problem]

The present invention is directed to providing a sulfate of a compound of Chemical Formula 1, which has excellent physicochemical properties, among various salts of a compound of Chemical Formula 1.

The present invention is also directed to providing various crystalline forms of a sulfate of a compound of Chemical Formula 1.

The present invention is also directed to providing a method of preparing a sulfate of a compound of Chemical Formula 1 and a method of preparing a crystalline form of a sulfate of a compound of Chemical Formula 1.

The present invention is also directed to providing a pharmaceutical composition including a sulfate of a compound of Chemical Formula 1 or a crystalline form thereof as an active ingredient.

The present invention is also directed to providing a use of a sulfate of a compound of Chemical Formula 1 or a crystalline form thereof.

### [Technical Solution]

One aspect of the present invention provides a sulfate of a compound of Chemical Formula 1, which has excellent physicochemical properties, among various salts of a compound of Chemical Formula 1.

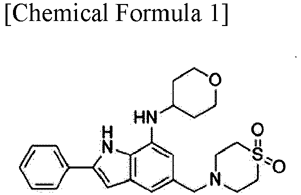

The sulfate of the compound of Chemical Formula 1 may be represented by the following Chemical Formula 2.

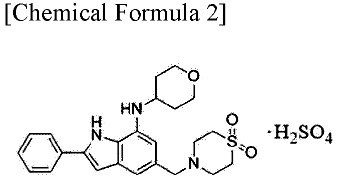

The compound of Chemical Formula 1 of the present invention may be referred to as "Compound 1."

In the present invention, various crystalline forms of the sulfate of the compound of Chemical Formula 1 are also identified, and 8 crystalline forms (crystalline forms I, II, III, IV, V, VI, VII and VIII) having different crystalline structures are identified.

Crystalline form I of the sulfate of the compound of Chemical Formula 1 may be identified with an X-ray powder diffraction pattern having at least 4 diffraction peaks, for example, at least 4, 5, 6, 7, 8, 9, 10 diffraction peaks, at 2[θ] values selected from among 6.63±0.2, 17.34±0.2, 17.76±0.2, 18.70±0.2, 18.93±0.2, 20.24±0.2, 20.83±0.2, 21.56±0.2, 24.66±0.2 and 29.00±0.2.

In particular, the X-ray powder diffraction pattern may have diffraction peaks at 2[θ] values selected from among 6.63±0.2, 17.34±0.2, 17.76±0.2, 18.93±0.2, 20.24±0.2 and 24.66±0.2.

Crystalline form II of the sulfate of the compound of Chemical Formula 1 may be identified with an X-ray powder diffraction pattern having at least 4 diffraction peaks, for example, at least 4, 5, 6, 7, 8, 9, 10 diffraction peaks, at 2[θ] values selected from among 6.57±0.2, 13.91±0.2, 16.05±0.2, 17.36±0.2, 18.41±0.2, 19.16±0.2, 19.36±0.2, 20.11±0.2, 20.77±0.2 and 24.31±0.2.

In particular, the X-ray powder diffraction pattern may have diffraction peaks at 2[θ] values selected from among 6.57±0.2, 17.36±0.2, 19.16±0.2, 19.36±0.2 and 20.11±0.2.

Crystalline form III of the sulfate of the compound of Chemical Formula 1 may be identified with an X-ray powder diffraction pattern having at least 4 diffraction peaks, for example, at least 4, 5, 6, 7, 8, 9, 10 diffraction peaks, at 2[θ] values selected from among 7.66±0.2, 16.32±0.2, 17.53±0.2, 18.05±0.2, 18.28±0.2, 18.95±0.2, 19.49±0.2, 19.91±0.2, 21.44±0.2, 22.24±0.2, 23.52±0.2, 24.29±0.2, 25.10±0.2, 27.37±0.2, 29.07±0.2 and 31.90±0.2.

In particular, the X-ray powder diffraction pattern may have diffraction peaks at 2[θ] values selected from among 7.66±0.2, 17.53±0.2, 18.28±0.2 and 19.91±0.2.

Crystalline form IV of the sulfate of the compound of Chemical Formula 1 may be identified with an X-ray powder diffraction pattern having at least 4 diffraction peaks, for example, at least 4, 5, 6, 7, 8, 9, 10 diffraction peaks, at 2[θ] values selected from among 6.30±0.2, 6.87±0.2, 8.58±0.2, 9.02±0.2, 10.26±0.2, 12.46±0.2, 14.71±0.2, 15.50±0.2, 17.42±0.2, 18.27±0.2, 19.56±0.2, 20.20±0.2, 21.55±0.2, 22.28±0.2 and 28.31±0.2.

In particular, the X-ray powder diffraction pattern may have diffraction peaks at 2[θ] values selected from among 6.30±0.2, 8.58±0.2, 9.02±0.2, 12.46±0.2, 15.50±0.2, 17.42±0.2, 19.56±0.2, 21.55±0.2 and 22.28±0.2.

Crystalline form V of the sulfate of the compound of Chemical Formula 1 may be identified with an X-ray powder diffraction pattern having at least 4 diffraction peaks, for example, at least 4, 5, 6, 7, 8, 9, 10 diffraction peaks, at 2[θ] values selected from among 6.88±0.2, 13.50±0.2, 16.17±0.2, 17.07±0.2, 17.10±0.2, 18.30±0.2, 18.54±0.2, 19.09±0.2, 19.71±0.2, 20.15±0.2, 20.52±0.2, 25.87±0.2 and 28.55±0.2.

In particular, the X-ray powder diffraction pattern may have diffraction peaks at 2[θ] values selected from among 6.88±0.2, 18.54±0.2, 19.09±0.2, 19.71±0.2 and 20.15±0.2.

Crystalline form VI of the sulfate of the compound of Chemical Formula 1 may be identified with an X-ray powder diffraction pattern having at least 4 diffraction peaks, for example, at least 4, 5, 6 diffraction peaks, at 2[θ] values selected from among 6.51±0.2, 15.73±0.2, 17.03±0.2, 19.01±0.2, 19.85±0.2, 20.45±0.2 and 24.20±0.2.

In particular, the X-ray powder diffraction pattern may have diffraction peaks at 2[θ] values selected from among 6.51±0.2, 17.03±0.2, 19.85±0.2 and 20.45±0.2.

Crystalline form VII of the sulfate of the compound of Chemical Formula 1 may be identified with an X-ray powder diffraction pattern having at least 3 diffraction peaks, for example, at least 3, 4, 5 diffraction peaks, at 2[θ] values selected from among 8.15±0.2, 16.30±0.2, 16.61±0.2 and 24.54±0.2.

In particular, the X-ray powder diffraction pattern may have diffraction peaks at 2[θ] values selected from among 16.30±0.2 and 16.61±0.2.

Crystalline form VIII of the sulfate of the compound of Chemical Formula 1 may be identified with an X-ray powder diffraction pattern having at least 4 diffraction peaks, for example, at least 4, 5, 6, 7, 8, 9, 10 diffraction peaks, at 2[θ] values selected from among 6.35±0.2, 6.78±0.2, 8.36±0.2, 9.40±0.2, 13.98±0.2, 15.98±0.2, 20.43±0.2, 21.15±0.2, 21.40±0.2 and 21.61±0.2.

In particular, the X-ray powder diffraction pattern may have diffraction peaks at 2[θ] values selected from among 6.35±0.2, 6.78±0.2, 8.36±0.2 21.15±0.2, 21.40±0.2 and 21.61±0.2.

Another aspect of the present invention provides a method of preparing a sulfate of a compound of Chemical Formula 1, which includes:
stirring a solution including a compound of the following Chemical Formula 1 and a solvent selected from ethanol, methyl ethyl ketone (MEK), ethyl acetate, methanol, and acetone under heating; and
drop-wise adding, to the solution, a solution including the same solvent as the solvent and sulfuric acid and performing cooling.

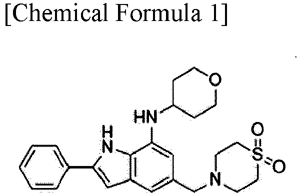

Still another aspect of the present invention provides a method of preparing Crystalline form III of a sulfate of the compound of Chemical Formula 1, which includes:
stirring a solution including the compound of Chemical Formula 1 and methanol under heating; and
drop-wise adding, to the solution, a solution including methanol and sulfuric acid and performing cooling.

In the methods of preparing a sulfate of the compound of Chemical Formula 1 and Crystalline form III thereof, the compound of Chemical Formula 1, which is used as a starting material, may be amorphous or in any crystalline form.

In the methods of preparing a sulfate of the compound of Chemical Formula 1 and Crystalline form III thereof, the heating may be performed by increasing a temperature up to 30 to 120 °C.

In addition, the cooling may be performed by lowering a temperature to 0 to 25 °C.

Meanwhile, in the methods of preparing a sulfate of the compound of Chemical Formula 1 and Crystalline form III thereof, the stirring may be performed for 30 minutes to 3 hours.

In addition, the dropwise addition may be performed for 1 to 8 hours, and the cooling may be performed for 5 to 30 hours.

In the method of preparing Crystalline form III of a sulfate of the compound of Chemical Formula 1, the solution including methanol may further include water, and a ratio of methanol and water may be 7:3 to 9.5:0.5, but the present invention is not limited thereto.

In the methods of preparing a sulfate of the compound of Chemical Formula 1 and Crystalline form III thereof, subsequent filtration or drying through which foreign substances, excess solvent, and the like are removed may be further performed to obtain a high-purity product.

Yet another aspect of the present invention provides a pharmaceutical composition including the above-described sulfate of the compound of Chemical Formula 1 or the crystalline form thereof, and a pharmaceutically acceptable carrier.

Yet another aspect of the present invention provides a use of the sulfate of the compound of Chemical Formula 1 or the crystalline form thereof in the prevention or treatment of diseases described below.

Yet another aspect of the present invention provides a method of preventing or treating diseases described below, which includes administering a pharmaceutically effective amount of the sulfate of the compound of Chemical Formula 1 or the crystalline form thereof to a subject in need thereof.

According to WO2009-025478, the compound of Chemical Formula 1 is reported to exhibit preventive, therapeutic and ameliorative effects against necrosis and associated diseases. According to WO2009-025478, necrosis and associated diseases are selected from the group comprised of acute/chronic hepatic disease (e.g. hepatitis, hepatic fibrosis, hepatocirrhosis), neurodegenerative disease (e.g. dementia, Parkinson's disease, Huntington's disease), ischemic cardiac disease, ischemic reperfusion injury (Korean Registered Patent 10-1325272), ischemic stroke or ischemic injury, pancreatitis, bacterial/viral sepsis, diabetes mellitus or diabetic complications, diabetic vascular disease [in particular, these diabetes are caused by pancreatic cell destroying substances, and mediated by virus, hyperglycemia, fatty acid, diet, toxin, streptozotocin and the like], necrotizing proctitis, cystic fibrosis, rheumatoid arthritis, degenerative arthritis, nephropathy, bacterial infection, viral infection (e.g. HIV), multiple sclerosis, leukemia, lymphoma, neonatal respiratory distress syndrome, asphyxia, tuberculosis, endometriosis, angiasthenia, psoriasis, chilblain, steroid treatment complications, gangrene, tenderness, hemoglobinuria, burn, hyperthermia, Crohn's disease, celiac disease, compartment syndrome, spinal cord injury, glomerulonephritis, muscular dystrophy, inherited metabolic disorder, mycoplasma disease, anthrax, Andersen's disease, congenital mitochondrial disease, phenylketonuria, placental infarction, syphilis, aseptic necrosis and the like. In addition, necrosis and associated diseases caused by drugs and toxic substances are selected from the group consisting of the necrosis associated with alcoholism, the exposure to, and/or administration and/or self-administration of, cocaine, drugs (e.g., paracetamol), antibiotics, anti-cancer agent, Adriamycin, puromycin, bleomycin, NSAID, cyclosporine, chemical toxins (e.g., carbon tetrachloride, cyanide, methanol, ethylene glycol), poison gas, agrochemicals, heavy metals (e.g., lead, mercury, cadmium), or injury due to the exposure to radioactivity/UV and associated necrosis thereof.

Further, the compound of Chemical Formula 1 is additionally expected to exhibit preventive, therapeutic and ameliorative effects in necrosis and the associated diseases of acute/chronic kidney disease, traumatic brain injury, the neurogenerative disease of amyotrophic lateral sclerosis (ALS), necrotizing colitis, viral infection (e.g. SARS-CoV), skin disease including psoriasis and allergic dermatitis, and organ preservation / organ transplantation (See Korean Registered Patents 10-1098583 and 10-1941004).

Further, a pharmaceutical composition comprising the compound of Chemical Formula 1 can regulate intracellular calcium and improve ER (Endoplasmic Reticulum) stress and mitochondrial dysfunction due to abnormal intracellular calcium levels. Accordingly, a pharmaceutical composition comprising the compound of Chemical Formula 1 is expected to exhibit preventive or therapeutic and ameliorative effects with regard to illnesses associated with the same. Associated illnesses are as follow.

Inflammatory pulmonary disease including acute lung injury syndrome / acute pulmonary disease, pneumonia, tuberculosis, asthma, pulmonary hypertension, chronic obstructive pulmonary disease, idiopathic pulmonary fibrosis, and cystic fibrosis (See Mitochondrial dysfunction in fibrotic diseases. Cell Death Discov. 2020 Sep 5; 6:80*.* Mitochondrial dysfunction in lung aging and diseases. Eur Respir Rev. 2020 Oct 15;29(157):200165*,* see Korean registered patent 10-1636563)

Demyelinating disease including demyelination and amyotrophic lateral sclerosis, hypertension including pulmonary hypertension, stroke, prion disease, epilepsy, ataxia, migraines, reduced cognitive skills, seizure, tremors, mental disorders (e.g., depression) (See Neuronal and glial calcium signaling in Alzheimer's disease. Cell Calcium. Oct-Nov 2003;34(4-5):385-97*.* Mitochondrial disorders: challenges in diagnosis & treatment. Indian J Med Res. 2015 Jan;141(1): 13-26*.)*

Insulin resistance, hyperlipidemia, atherosclerosis, inflammatory bowel disease (IBD) including Crohn's Disease and ulcerative colitis, various cancers and metastasis of cancer (See reticulum stress and oxidative stress in cell fate decision and human disease. Antioxid Redox Signal. 2014 Jul 20;21(3):396-413*.)*

Visual impairment-associated disease (e.g., retinitis pigmentosa, optic neuropathy, cataract, glaucoma), anemia, cholestasis, hypoparathyroidism, pancytopenia, pancreatic disorder, lactic acidosis, lactacidemia, loss of hearing, short stature, intestinal obstruction, cardiac conduction defect, cardiomyopathy, endometriosis, infertility, early menopause (See Mitochondrial diseases: the contribution of organelle stress responses to pathology. Nat Rev Mol Cell Biol. 2018 Feb;19(2):77-92*.* Seminars in medicine of the Beth Israel Hospital, Boston. Mitochondrial DNA and disease. N Engl J Med. 1995 Sep 7;333(10):638-44*.* Mitochondrial injury and dysfunction in hypertension-induced cardiac damage. Eur Heart J. 2014 Dec 7; 35(46): 3258-3266*.*

Muscular atrophy diseases including limb gridle/Becker muscular dystrophy (LGMD/BMD) and Duchenne muscular dystrophy (DMD) (Duchenne muscular dystrophy is associated with the inhibition of calcium uniport in mitochondria and an increased sensitivity of the organelles to the calcium-induced permeability transition. See Biochim Biophys Acta Mol Basis Dis. 2020 May 1;1866(5):165674*.*)

Aging and aging-related diseases (See Interrelation between ROS and Ca2+ in aging and age-related diseases. Redox Biology. 2020; 6:101678*.*)*.*

According to WO2009-025478, a pharmaceutical composition comprising the compound of Chemical Formula 1 exhibits not only liver protection and liver function improvement effects, but also preventive and therapeutic effects against acute and chronic liver diseases such as fatty liver, hepatic fibrosis, liver cirrhosis, and virus or drug-induced hepatitis. Further, as a result the pharmaceutical composition may prevent or treat liver disease complications such as portal hypertension but is not limited to such. Even further, the pharmaceutical composition according to the present invention is effective in preventing and treating liver disease selected from among liver transplant, alcoholic or non-alcoholic fatty liver disease (See Korean Registered Patent 10-2006247), hepatic fibrosis, liver cirrhosis, and virus or drug-induced hepatitis, and is effective against acute and chronic alcoholic liver disease. Further, the composition according to the present invention is effective in treating or preventing fatty liver caused by fatty acids or acute or chronic liver disease caused by fatty liver.

According to Korean Registered Patent No. 10-1852304, the compound of Chemical Formula 1 may be used in a stem cell culture to improve the differentiation efficiency and maturity of stem cell-derived cardiomyocytes.

Further, according to WO2016-072692, the compound of Chemical Formula 1 may be used to prevent and treat mucositis.

In the present invention, "treatment" means the interrupting or delaying the progress of the disease when applied to the subject showing the onset of disease symptoms, and "prevention" means the interrupting or delaying the sign of the onset of disease when applied to the subject that does not show, but is at risk of, the onset of disease symptoms.

In the present invention, "pharmaceutical composition" may comprise pharmaceutically acceptable carriers, together with the compounds of the present invention.

The compound of Chemical Formula 1 according to the present invention may be administered in various oral and non-oral dosage forms for clinical administration, and when formulated, is prepared using diluents or excipients such as commonly used fillers, bulking agents, binding agents, wetting agents, disintegrating agents, or surfactants.

Solid formulations for oral administration include tablets, pills, powders, granules, capsules and troches, and such solid formulations are prepared by mixing at least one of the compounds of the present invention with at least one excipient, for example, starch, calcium carbonate, sucrose, lactose, or gelatin. Further, in addition to simple excipients, lubricating agents such as magnesium stearate, or talc is used. Liquid formulations for oral administration include suspensions, liquids for internal use, emulsions, or syrups, and in addition to the commonly used simple diluents water and liquid paraffin, various excipients, for example wetting agents, sweeteners, aromatics, and preservatives may be included.

Formulations for non-oral administration include sterile aqueous solutions, non-aqueous solvents, suspensions, emulsions, lyophilized preparations, suppositories. As the non-aqueous solvent and the suspension solvent, propylene glycol, polyethylene glycol, vegetable oil such as olive oil, injectable ester such as ethyl oleate, and the like can be used. As the base of the suppository, witepsol, macrogol, tween 61, cacao butter, laurin butter, glycerol, gelatin and the like can be used.

Further, the effective human dose for the compound of Chemical Formula 1, the sulfate thereof, or the crystalline form of thereof according to the present invention may vary depending on the age, body weight, sex, mode of administration, health status and severity of illness of the patients, and is normally approximately 0.001-100mg/kg/day, preferably 0.01-35 mg/kg/day. For an adult patient with a body weight of 70kg, it is normally 0.07-7000mg/day, preferably 0.7-2500mg/day, and depending on the judgment of a physician or pharmacist it may be administered once a day or divisionally across multiple administrations at certain time intervals.

### [Advantageous Effects]

The sulfate of the compound of Chemical Formula 1 according to the present invention has much higher solubility than other salts thereof and exhibits excellent stability and absorption into the body. In addition, the sulfate can form various crystalline forms, and these crystalline forms also are stable and undergo almost no reduction in purity even when stored under various conditions for a long period of time, and thus are useful for storage.

### [Description of Drawings]

FIGS. 1 to 13 show X-ray powder diffraction (XRPD) results of a compound of Chemical Formula 1 for each counter ion under 4 types of solvents (acetone, THF, DMSO and IPA/H₂O).
FIG. 14 shows a differential scanning calorimetry (DSC)/thermogravimetric analysis (TGA) result of a hydrochloride of Compound 1, FIG. 15 shows a DSC/TGA result of a sulfate thereof, FIG. 16 shows a DSC/TGA result of a methanesulfonate thereof, FIG. 17 shows a DSC/TGA result of a p-toluenesulfonate thereof, and FIG. 18 shows a DSC/TGA result of a maleate thereof.
FIG. 19 shows a high-performance liquid chromatography (HPLC) spectrum of a free form of Compound 1, as measured in a physicochemical stability test, FIG. 20 shows an HPLC spectrum of the hydrochloride of Compound 1, FIG. 21 shows an HPLC spectrum of the sulfate, FIG. 22 shows an HPLC spectrum of the methanesulfonate, FIG. 23 shows an HPLC spectrum of the p-toluenesulfonate, and FIG. 24 shows an HPLC spectrum of the maleate.
FIG. 25 shows an XRPD result of the free form of Compound 1, as measured in a physicochemical stability test, FIG. 26 shows an XRPD result of the hydrochloride of Compound 1, FIG. 27 shows an XRPD result of the sulfate, FIG. 28 shows an XRPD result of the methanesulfonate, FIG. 29 shows an XRPD result of the p-toluenesulfonate, and FIG. 30 shows an XRPD result of the maleate.
FIG. 31 shows the pharmacokinetic test results of the compound of Chemical Formula 1 and 5 types of salts thereof.
FIG. 32 shows the XRPD results of the sulfate of Compound 1 produced under 5 types of solvents.
FIG. 33 shows a DSC/TGA result of the sulfate of Compound 1 produced under an ethanol solvent, FIG. 34 shows a DSC/TGA result of the sulfate produced under a methyl ethyl ketone solvent, FIG. 35 shows a DSC/TGA result of the sulfate produced under an ethyl acetate solvent, FIG. 36 shows a DSC/TGA result of the sulfate produced under a methanol/water (9/1) solvent, FIG. 37 shows a DSC/TGA result after the sulfate produced under a methanol/water (9/1) solvent is dried at 50 °C for 5 days, and FIG. 38 shows a DSC/TGA result of the sulfate produced under an acetone/water (9/1) solvent.
FIG. 39 shows an XRPD result of the sulfate of Compound 1 produced by a slurry method (25 °C, 1 week) of Example 5-1, FIG. 40 shows an XRPD result of the sulfate of Compound 1 produced by a slurry method (50 °C, 1 week), FIG. 41 shows an XRPD result of the sulfate of Compound 1 produced by a slurry method (25 °C, 4 weeks), and FIG. 42 shows an XRPD result of the sulfate of Compound 1 produced by a slurry method (50 °C, 4 weeks).
FIG. 43 shows an XRPD result of the sulfate of Compound 1 produced by a slow evaporation method of Example 5-2.
FIG. 44 shows an XRPD result of the sulfate of Compound 1 according to a heat-cooling method of Example 5-3.
FIG. 45 shows an XRPD result of the sulfate of Compound 1 according to an anti-solvent method of Example 5-4.
FIG. 46 shows a DSC result of the sulfate of Compound 1 produced by crystallization method 1 of Example 5-6.
FIG. 47 shows a DSC result of the sulfate of Compound 1 produced by crystallization method 2 of Example 5-6.
FIG. 48 shows an XRPD result of the sulfate of Compound 1 produced by crystallization methods 1 and 2 of Example 5-6.
FIG. 49 shows a dynamic vapor sorption (DVS) result of the sulfate of Compound 1 of Example 5-7, and FIG. 50 shows an XRPD result of the sulfate of Compound 1 of Example 5-7 after a DVS test.
FIG. 51 shows an XRPD result of the sulfate of Compound 1 according to a water activity test of Example 5-8.
FIG. 52 shows an XRPD result of the sulfate of Compound 1 according to a compressive behavior test of Example 5-9.
FIG. 53 shows an XRPD result of the sulfate of Compound 1 according to a grinding method of Example 5-10.
FIG. 54 shows an XRPD result of Crystalline form I of the sulfate of Compound 1.
FIG. 55 shows an HPLC spectrum of Crystalline form I of the sulfate of Compound 1.
FIG. 56 shows a TGA/DSC graph of Crystalline form I of the sulfate of Compound 1, and FIG. 57 shows an XRPD result of Crystalline form I before and after TGA.
FIG. 58 shows an XRPD result of Crystalline form II of the sulfate of Compound 1.
FIG. 59 shows an HPLC spectrum of Crystalline form II of the sulfate of Compound 1.
FIG. 60 shows a TGA/DSC graph of Crystalline form II of the sulfate of Compound 1, and FIG. 61 shows an XRPD result of Crystalline form II of the sulfate of Compound 1 before and after TGA.
FIG. 62 shows an XRPD result of Crystalline form III of the sulfate of Compound 1.
FIG. 63 shows an HPLC spectrum of Crystalline form III of the sulfate of Compound 1.
FIG. 64 shows a TGA/DSC graph of Crystalline form III of the sulfate of Compound 1, FIG. 65 shows an ¹H-NMR spectrum of Crystalline form III of the sulfate of Compound 1, and FIG. 66 shows an XRPD result of Crystalline form III before and after TGA.
FIG. 67 shows an XRPD result of Crystalline form IV of the sulfate of Compound 1.
FIG. 68 shows an HPLC spectrum of Crystalline form IV of the sulfate of Compound 1. FIG. 69 shows a TGA/DSC graph of Crystalline form IV of the sulfate of Compound 1.
FIG. 70 shows an XRPD result of Crystalline form V of the sulfate of Compound 1.
FIG. 71 shows an HPLC spectrum of Crystalline form V of the sulfate of Compound 1, FIG. 72 shows a TGA/DSC graph of Crystalline form V of the sulfate of Compound 1, and FIG. 73 shows an XRPD result of Crystalline form V of the sulfate of Compound 1 before and after TGA.
FIG. 74 shows an XRPD result of Crystalline form VI of the sulfate of Compound 1.
FIG. 75 shows an HPLC spectrum of Crystalline form VI of the sulfate of Compound 1, FIG. 76 shows a TGA/DSC graph of Crystalline form VI of the sulfate of Compound 1, and FIG. 77 shows an XRPD result of Crystalline form VI of the sulfate of Compound 1 before and after TGA.
FIG. 78 shows an XRPD result of Crystalline form VII of the sulfate of Compound 1.
FIG. 79 shows an HPLC spectrum of Crystalline form VII of the sulfate of Compound 1.
FIG. 80 shows an XRPD result of Crystalline form VIII of the sulfate of Compound 1, and FIG. 81 shows an XRPD result of Crystalline form VIII of the sulfate of Compound 1.
FIG. 82 shows an XRPD result of Crystalline form II of the sulfate of Compound 1 scaled up to 200 mg, and FIG. 83 shows a TGA/DSC result thereof.
FIG. 84 shows an XRPD result of Crystalline form III of the sulfate of Compound 1 scaled up to 10 g, and FIG. 85 shows a TGA/DSC result thereof.
FIG. 86 shows an XRPD result of Crystalline form IV of the sulfate of Compound 1 scaled up to 500 mg, FIG. 87 shows an HPLC spectrum thereof, and FIG. 88 shows a TGA/DSC result thereof.
FIG. 89 shows an XRPD result of Crystalline form VI of a sulfate of Compound 1 scaled up to 300 mg, and FIG. 90 shows a TGA/DSC result thereof.
FIG. 91 shows an XRPD change of Crystalline form III of a sulfate of Compound 1 during a stability test.
FIG. 92 shows an HPLC change of Crystalline form III of a sulfate of Compound 1 during a stability test.

### [Modes of the Invention]

The advantages and features of the present invention and the methods for achieving the advantages and features will become apparent with reference to the examples described below in detail. However, the present invention is not limited to the examples described herein and may be embodied in other forms. Rather, the examples have been provided to make the disclosure of the present invention thorough and complete and to fully inform the scope of the present invention to those of ordinary skill in the art to which the present invention pertains, and the present invention is defined only by the scope of the claims.

### [Examples]

### Abbreviations

The following abbreviations are used in this application.

| | |
|---|---|
| MeOH | Methanol |
| EtOH | Ethanol |
| DMSO | Dimethyl sulfoxide |
| NMP | N-methylpyrrolidinone |
| DMF | N,N-dimethylformamide |
| MTBE | Methyl t-butyl ether |
| MIBK | Methyl isobutyl ketone |
| ACN | Acetonitrile |
| MEK | Methyl ethyl ketone |
| IPA | Isopropanol |
| EtOAc | Ethyl acetate |
| RPM | Rotations per minute |
| TGA/DSC | Thermogravimetric analysis/differential scanning calorimetry |
| RH | Relative humidity |

### Used instruments, standards and methodologies

### 1. X-ray powder diffraction (XRPD)

Measurement was made using a Bruker D8 Advance powder diffractometer. An X-ray diffraction pattern was recorded while rotating a sample 3° to 40° using Cu K-alpha X-ray (1=1.54179 Å) radiation (40 kV/40 mA).
Rotational speed of sample = 15 rpm
Scanning rate = 18.5°/min

### 2. Differential scanning calorimetry (DSC)

Measurement was made using TA DSC Q2000 and Discovery DSC-2500 by placing about 1 mg of a sample in a hermetic aluminum pan with pinholes and increasing a temperature from 25 °C to 300 °C at 10 °C/min.

### 3. Thermogravimetric analysis (TGA)

Measurement was made using TGA Q50 or TA Q5000 by placing about 4 mg of a sample in an open platinum pan and increasing a temperature from 30 °C to 300 °C at 10 °C/min.

### 4. Particle size distribution (PSD) analysis

Measurement was made using a Sympatec HELOS particle size analyzer with a dispersing system using RODOS at a pressure of 0.5 bar.

### 5. Polarized light microscopy (PLM)

A 5-megapixel CCD Nikon LV100POL microscope with a 20x physical lens was used.

### 6. Water sorption and desorption isotherm analysis (DVS cycle)

10 mg of a sample was placed in a mesh stainless steel basket using SMS DVS Advantage 1. The entire experimental cycle consists of two scans (water sorption and desorption) at a constant temperature (25 °C) and 10% RH intervals in the range of 40 to 90% (60 to 360 minutes for each humidity level).

### Example 1: Preparation of 5-[(1,1-dioxido-4-thiomorpholinyl)methyl]-2-phenyl-N-(tetrahydro-2H-pyran-4-yl)-1H-indol-7-amine

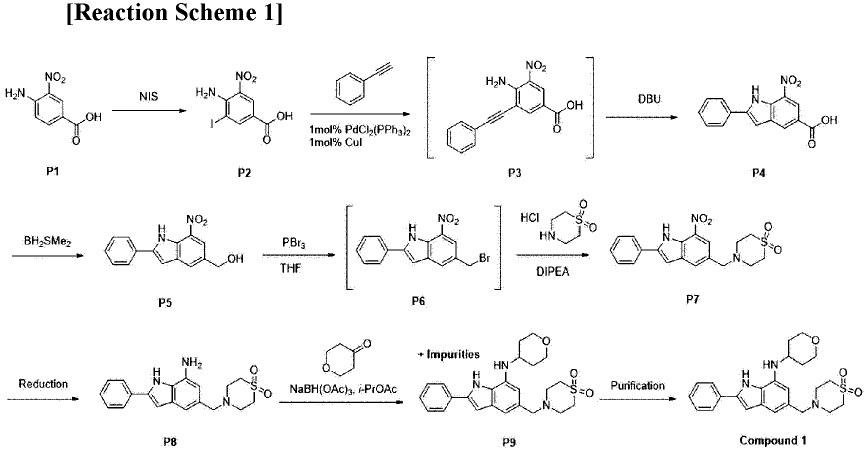

A method of preparing a compound of Chemical Formula 1, which is published in WO2009-025478, is represented by Reaction Scheme 1. P9 prepared according to Reaction Scheme 1 contains a large amount of impurities and foreign substances. Since the compound of Chemical Formula 1 has very low solubility, a purification process becomes very important. In order to remove the foreign substances and color of P9 initially obtained for research, P9 was dissolved in DMSO and filtered, and EtOH was added as an anti-solvent to obtain the compound of Chemical Formula 1. Also, the pure form of the compound of Chemical Formula 1, which was not subjected to any modification, was described as a "free form" and distinguished from salts or crystalline forms.

The prepared compound of Chemical Formula 1 exhibited very low solubility in not only water but also various solvents as shown in the following Table 1. Very low solubility in various solvents adversely affects pharmaceutical development that requires the production of a finished product in a completely dissolved state.

Therefore, the present invention deals with a salt of the compound of Chemical Formula 1 in a physicochemically stable form and a crystalline form thereof based on research on the compound of Chemical Formula 1 in a salt form for improving the solubility of the compound of Chemical Formula 1.

**[Table 1]**

| Solvent | Solubility (mg/mL) | | Solvent | Solubility (mg/mL) | |
|---|---|---|---|---|---|
| | 25 °C | 50 °C | | 25 °C | 50 °C |
| Methanol (MeOH) | 0.59 | 1.15 | Tetrahydrofuran (THF) | 17.40 | 16.26 |
| Ethanol (EtOH) | 1.41 | 1.08 | 2-Methyltetrahydrofuran (2-MeTHF) | 2.78 | 3.41 |
| Isopropanol (IPA) | 0.32 | 0.54 | N-methyl pyrrolidone (NMP) | 160.03 | 192.42 |
| 1-Butanol | 0.65 | 1.00 | Dimethyl sulfoxide (DMSO) | 43.35 | 238.15 |
| Acetonitrile (ACN) | 3.09 | 4.04 | Chloroform (CHCl₃) | 4.70 | 4.62 |
| Acetone | 5.29 | 17.50 | Toluene | 0.02 | 0.02 |
| Methyl ethyl ketone (MEK) | 6.08 | 8.12 | Heptane | <LOQ | <LOQ |
| Methyl isobutyl ketone (MIBK) | 2.10 | 2.82 | Water | 0.001 | 0.046 |
| Ethyl acetate (EtOAc) | 1.36 | 1.86 | MeOH/water=1:1 (v/v) | 0.03 | 0.08 |
| Isopropyl acetate (iPrOAc) | 0.71 | 0.91 | Acetone-water (1:2) | 0.08 | 0.16 |
| Methyl tert-butyl ether (MTBE) | 0.06 | 0.07 | Acetic acid-water (1:1) | 52.77 | 75.61 |
| N,N-dimethylformamide (DMF) | 206.03 | 332.99 | - | | |

### Example 2: Solvent and salt screening for compound of Chemical Formula 1

### Example 2-1: Selection of counter ion and solvent

Based on the pKa (4.50; UV-metric method) of the compound of Chemical Formula 1, 12 acids were selected as counter ions for salt screening research, and acetone, THF, DMSO and isopropanol/water (95/5, v/v) were selected as salt screening solvents. As shown in the following Table 2, each combination of counter ions and solvents were given a batch number.

**[Table 2]**

| **Batch numbers for counter ions and solvents** | | | | |
|---|---|---|---|---|
| Counter ions | Acetone | THF | DMSO | IPA/H₂O (95/5, v/v) |
| Control | 0A | 0B | 0C* | 0D |
| Hydrochloric acid | 1A | 1B* | 1C* | 1D* |
| Sulfuric acid | 2A | 2B | 2C* | 2D |
| Phosphoric acid | 3A | 3B* | 3C* | 3D |
| Methane sulfonic acid | 4A | 4B | 4C* | 4D |
| Acetic acid | 5A | 5B | 5C* | 5D |
| p-Toluenesulfonic acid monohydrate | 6A | 6B | 6C* | 6D* |
| Maleic acid | 7A | 7B | 7C* | 7D |
| L(+)-tartaric acid | 8A | 8B | 8C* | 8D |
| Fumaric acid | 9A | 9B | 9C* | 9D |
| Citric acid | 10A | 10B | 10C* | 10D |
| L-malic acid | 11A | 11B | 11C* | 11D |
| Succinic acid | 12A | 12B | 12C* | 12D |

| | | | | |
|---|---|---|---|---|
| * Addition of anti-solvent for solid production | | | | |

### Example 2-2: Salt screening

Salt screening was performed by the following procedure, and a total of 52 (4×13) screening experiments were conducted simultaneously.

In the case of solid counter ions such as p-toluenesulfonic acid, maleic acid, L-tartaric acid, fumaric acid, citric acid, L-malic acid, succinic acid and the like, 50 mg of the compound of Chemical Formula 1 and 1 mL of a solvent containing 1.1 equivalents of counter ions were input into a 2 mL vial. In the case of liquid counter ions rather than the solid counter ions, 50 mg of the compound of Chemical Formula 1 was input into a 2 mL vial, and 749 µL of a solvent was input into the vial. Then, a solution (251 µL, concentration: 0.5 mol/L) containing 1.1 equivalents of counter ions was input into the vial. Also, 1 mL was added, and 50 mg of the compound of Chemical Formula 1 was used as a control for determining whether salt formation occurred or not.

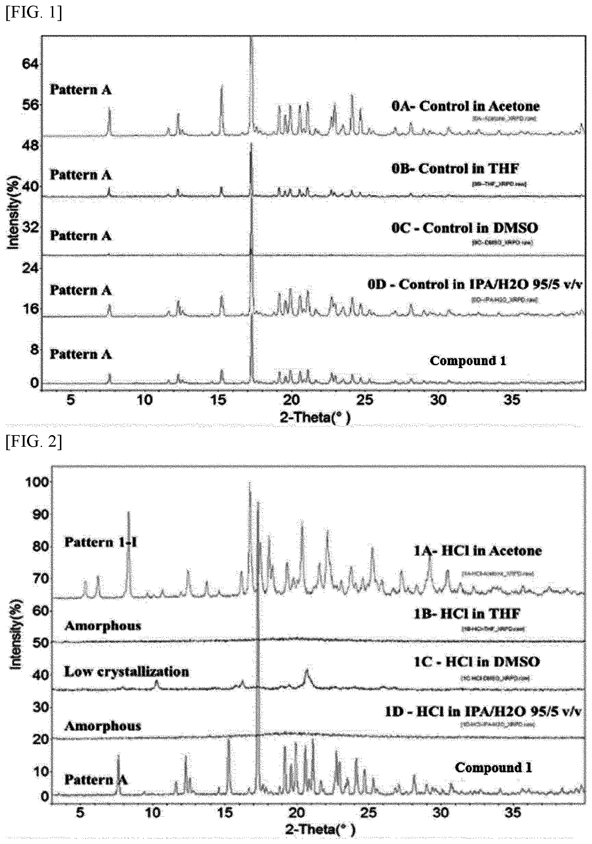

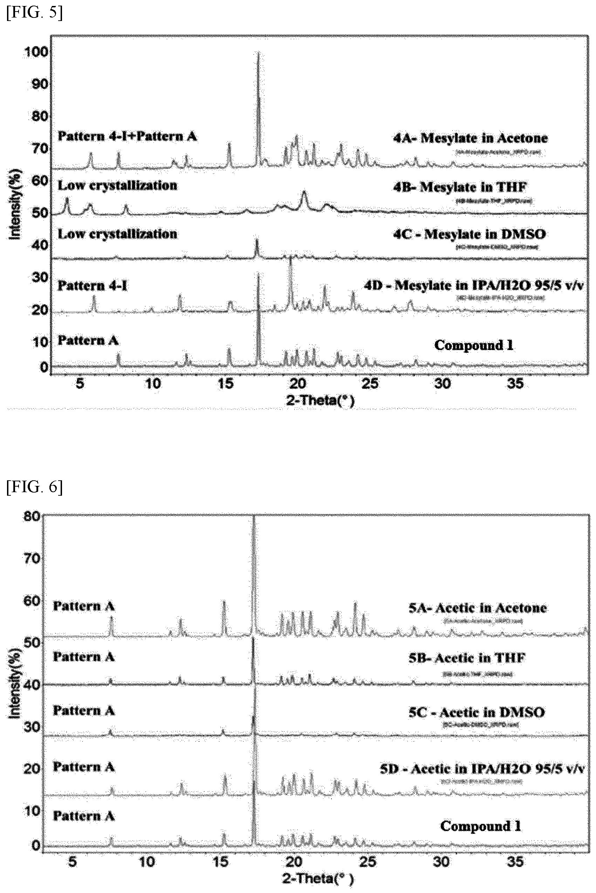

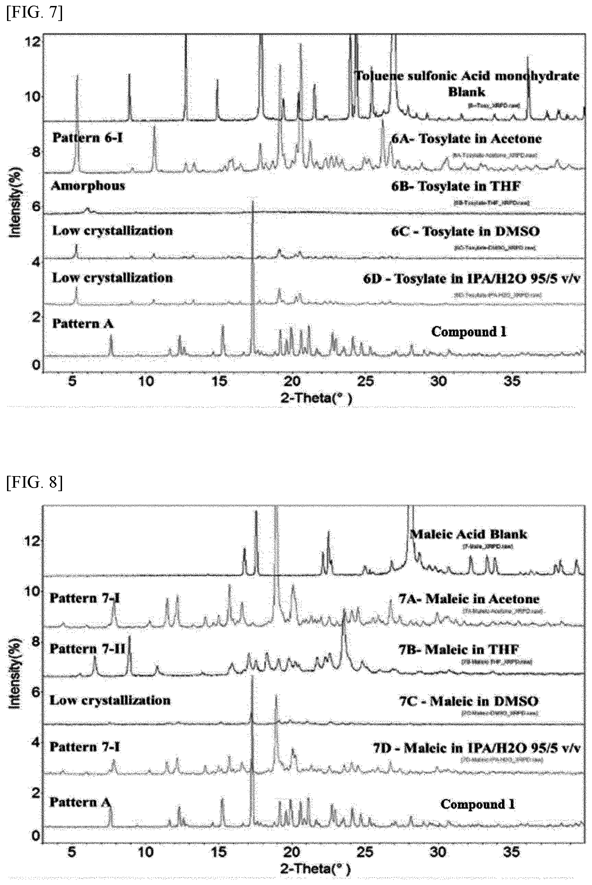

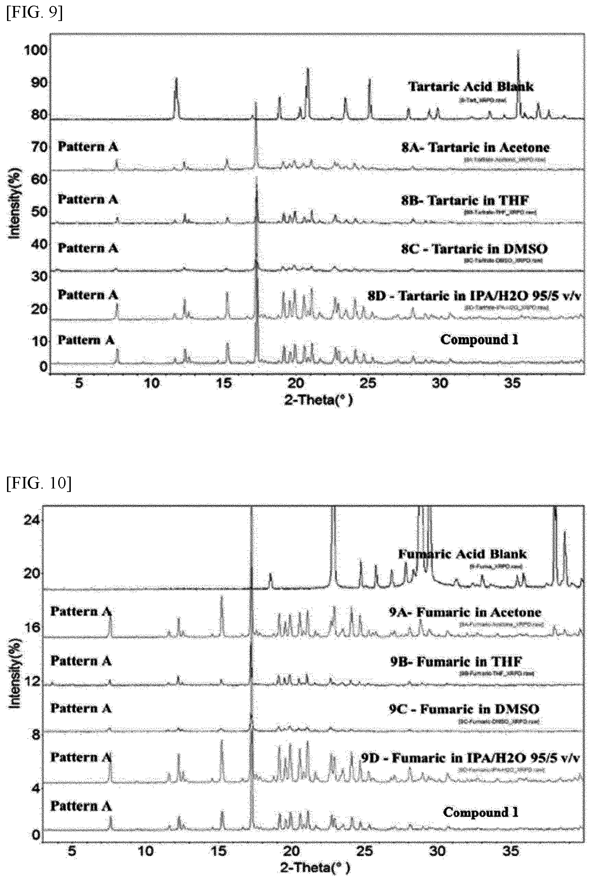

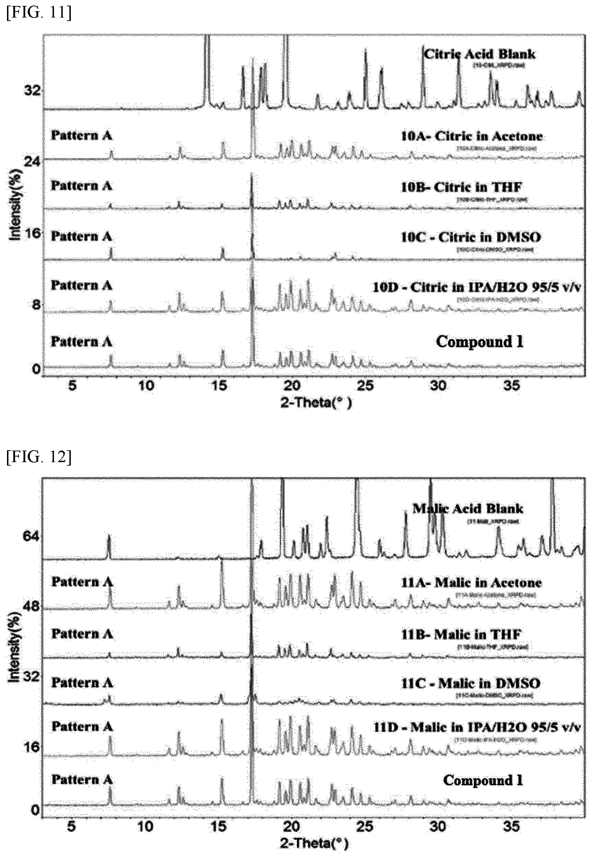

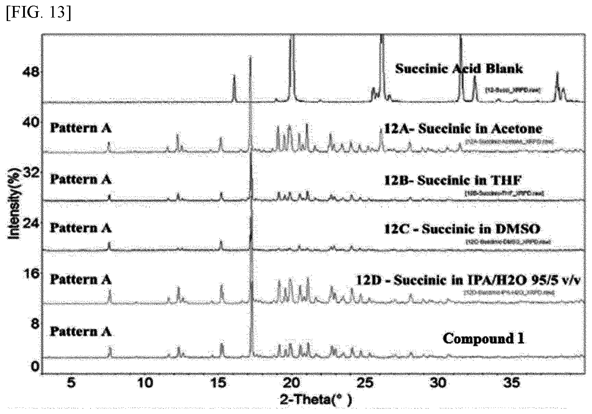

All experimental vials were placed on a high- and low-temperature cooling circulator with a stirring bar, heated to 50 °C, stirred at 900 rpm for 16 hours, and then cooled at 4 °C for 75 minutes. Afterward, the solid content of the suspension was collected by centrifugation and dried in a vacuum oven at 40 °C for 2 days, and then whether salt formation succeeded or not was assessed by PLM and XRPD methods. In the case of the complete dissolution system, the solid content was artificially produced by separately adding an anti-solvent, centrifuged, and dried in a vacuum oven for 2 days, and then whether salt formation succeeded or not was assessed by PLM and XRPD methods. FIGS. 1 to 13 and Table 3 show XRPD results for each counter ion under 4 types of solvents of Table 2.

**[Table 3]**

| | | | | | |
|---|---|---|---|---|---|
| **PLM and XRPD experiment results for salts obtained in 4 types of solvents** | | | | | |

| No. | Counter ions | Acetone | THF | DMSO | IPA/H₂O (95/5, v/v) |
|---|---|---|---|---|---|
| Con. | None | S-S*, Pattern A | S-S, Pattern A | H-C, Pattern A | S-S, Pattern A |
| 1 | Hydrochloric acid | H-S, Pattern 1-1 | C with wax-C with wax, Amorphous^{eva} | H-C, LC^{eva} | H-H, Amorphous^{eva} |
| 2 | Sulfuric acid | S-S, LC | S-S, Amorphous | H-C, Amorphous^{eva} | S-S, Pattern 2-1 |
| 3 | Phosphoric acid | S-S, Pattern A | C with wax-C, Pattern A^{eva} | H-C, Pattern A^{eva} | S-S, Pattern A |
| 4 | Methane sulfonic acid | S-S, Pattern (4-I)+A | S-S, LC | H-C, LC^{eva} | S-S, Pattern 4-1 |
| 5 | Acetic acid | S-S, Pattern A | S-S, Pattern A | H-C, Pattern A^{eva} | S-S, Pattern A |
| 6 | Para-Toluene sulfonic acid | H-S, Pattern 6-1 | C-S, Amorphous | H-C, LC ^{eva} | H-C with wax, LC^{eva} |
| 7 | Maleic acid | S-S, Pattern 7-1 | C-S, Pattern 7-II | H-C, LC^{eva} | S-S, Pattern 7-1 |
| 8 | L(+)-Tartaric acid | S-S, Pattern A | S-S, Pattern A | H-C, Pattern A^{eva} | S-S, Pattern A |
| 9 | Fumaric acid | S-S, Pattern A | S-S, Pattern A | H-C, Pattern A^{eva} | S-S, Pattern A |
| 10 | Citric acid | S-S, Pattern A | S-S, Pattern A | H-C, Pattern A^{eva} | S-S, Pattern A |
| 11 | L-Malic acid | S-S, Pattern A | S-S, Pattern A | H-C, Pattern A^{eva} | S-S, Pattern A |
| 12 | Succinic acid | S-S, Pattern A | S-S, Pattern A | H-C, Pattern A^{eva} | S-S, Pattern A |

| | | | | | |
|---|---|---|---|---|---|
| · * Observation: initial → final · Roman numerals: different crystalline patterns of the same salt · Pattern A: pattern of compound of Chemical Formula 1 · S: suspension · C: complete dissolution · H: suspension · LC: low crystallinity · eva: solid sample obtained after addition of anti-solvent | | | | | |

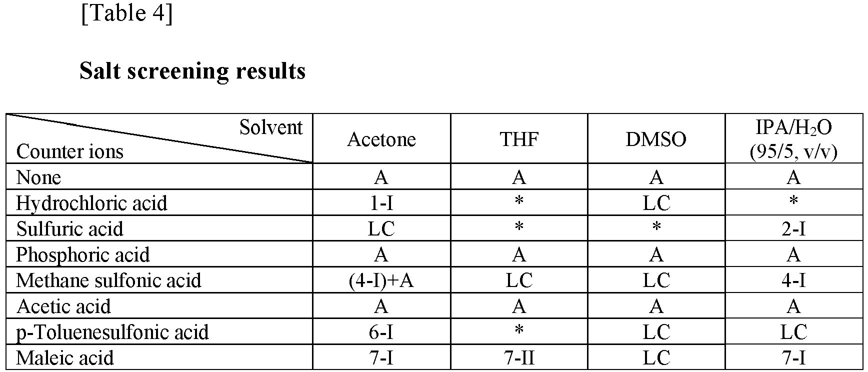

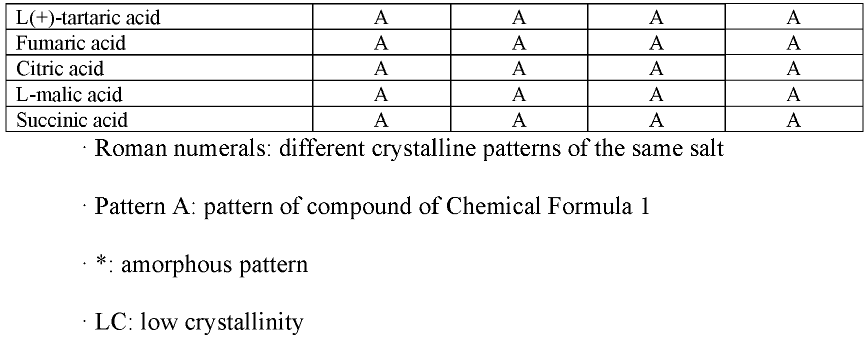

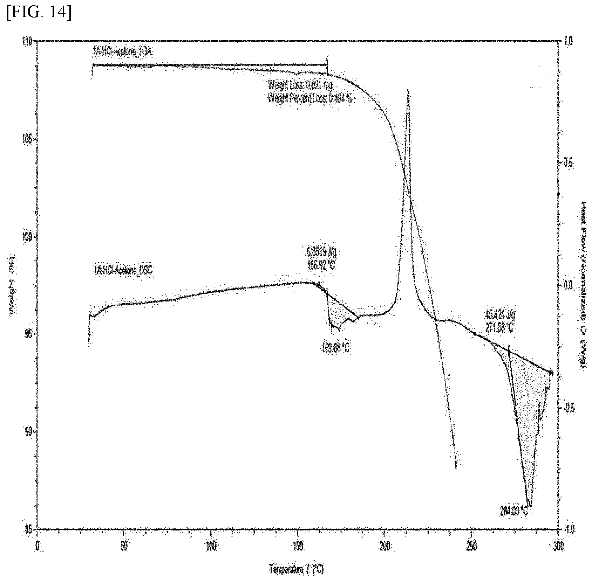

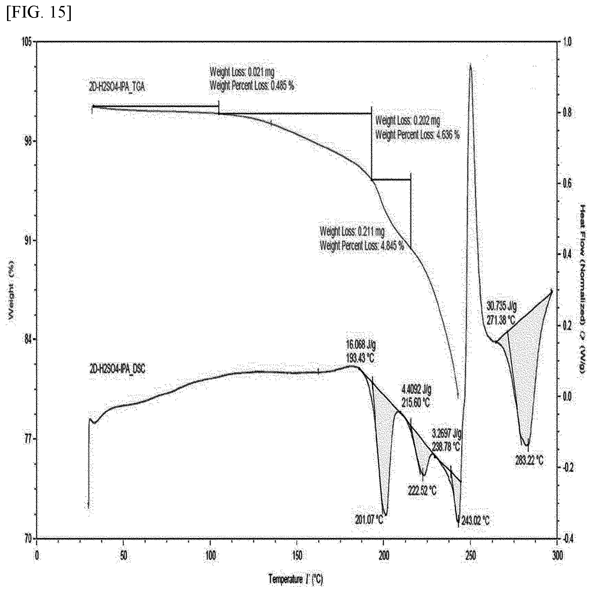

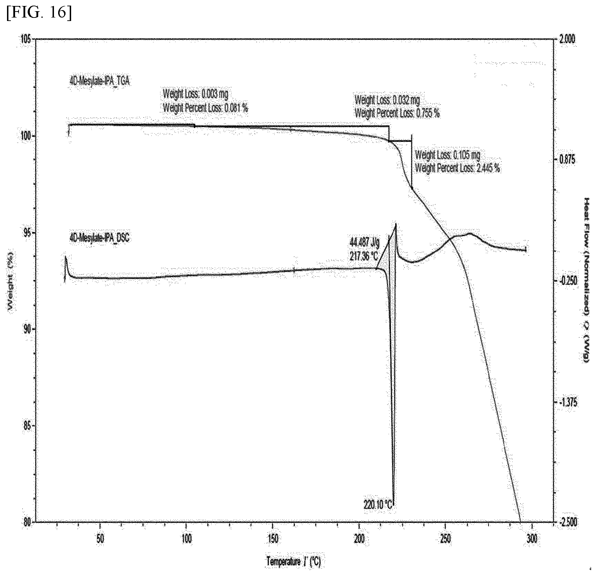

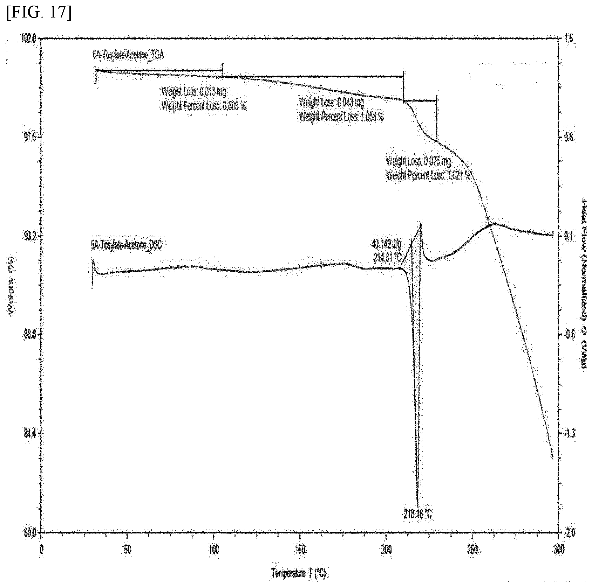

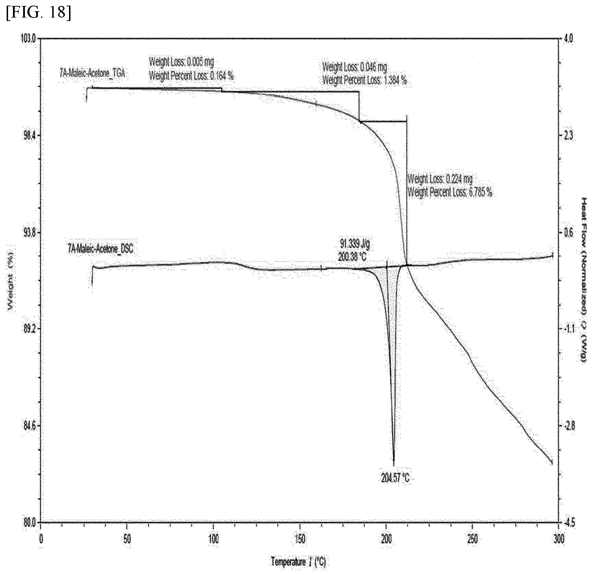

As shown in Tables 3 and 4, as a result of salt screening, 5 potential salt compounds of the compound of Formula 1 with a total of 6 novel crystalline forms, hydrochloride (Pattern 1-I), sulfate (Pattern 2-I), methanesulfonate (Pattern 4-I), p-toluenesulfonate (Pattern 6-I), and maleate (Patterns 7-I and 7-II), were found. FIGS. 14 to 18 and Table 5 show the results of additional DSC and TGA experiments for the 5 salt compounds of the compound of Chemical Formula 1.

**[Table 5]**

| **Identification of characteristics of 5 salt candidates of compound of Chemical Formula 1** | | | | | | |
|---|---|---|---|---|---|---|
| | Compound 1 | Hydrochlor ide | Sulfate | Mesylate | Tosylate | Maleate |
| Batch No. | | 1A | 2D | 4D | 6A | 7A |
| XRPD | A | 1-1 | 2-1 | 4-1 | 6-1 | 7-1 |
| Crystallinit y (XRD) | High | High | High | High | High | High |
| Melting Point (DSC, °C) | 274 | 167, 272 | Multiple peaks+ 193, 271 | 217 | 215 | 200 |
| Enthalpy (DSC, J/g) | 120.73 | 6.85, 45.42 | 16.07, 30.74 | 44.49 | 40.14 | 91.34 |
| Weight loss (TGA, %) | 0.038% <105 °C | 0.494% <167 °C | 0.485% <105 °C | 0.081% <105 °C | 0.306% <105 °C | 0.164% <105 °C |

### Example 3: Assessment of characteristics of salt compounds of compound of Chemical Formula 1

Characteristics of the hydrochloride, sulfate, methanesulfonate, p-toluenesulfonate and maleate of the compound of Chemical Formula 1 of Example 2 were confirmed through simulated gastrointestinal fluid solubility, physicochemical stability and pharmacokinetic tests as described below.

### Example 3-1: Simulated gastrointestinal fluid solubility test

In order to assess the dissolution behavior of the salt compounds of the compound of Chemical Formula 1 of the present invention, pH-dependent solubility was assessed in the simulated gastrointestinal tract. As simulated gastrointestinal fluid media (Biorelevant dissolution media) used for environment assessment in the simulated gastrointestinal tract, simulated gastric fluid (SGF), fasted state simulated intestinal fluid (FaSSIF), and fed state simulated intestinal fluid (FeSSIF) were used, and a detailed description of the test is as follows.

About 5 mg of 5 types of salts of the compound of Chemical Formula 1 was each divided into 3 vials of 2 mL capacity, and then 1 mL of the simulated gastrointestinal fluid medium (SGF, FaSSIF or FeSSIF) was added to each vial. All vials were placed on a hot plate stirrer and maintained while stirring at 37 °C and 700 rpm. When the compound was completely dissolved in the simulated gastrointestinal fluid medium, each salt of the compound of Chemical Formula 1 was further added until a suspension was formed. When the concentration of the dissolved salt of the compound of Chemical Formula 1 exceeded 10 mg/mL, no more salt was added. After mixing at 37 °C for 24 hours, 300 µL of the suspension was separated from each vial and analyzed.

The analysis sample was centrifuged at 14000 rpm for 5 minutes, and then the supernatant was diluted 10-fold with ACN:H₂O (7/3, v/v) and analyzed by ultra-performance liquid chromatography (UPLC). The solubility test results including the final pH of the simulated gastrointestinal fluid were summarized in Table 6.

**[Table 6]**

| **Final solubility and pH results after 24 hours in simulated gastrointestinal fluid** | | | | | | |
|---|---|---|---|---|---|---|
| Salts of compound of Chemical Formula 1 | Solubility (mg/mL) / pH | | | | | |
| | SGF (pH=1.80) | | FaSSIF (pH=6.53) | | FeSSIF (pH=4.98) | |
| | Solubility | pH | Solubility | pH | Solubility | pH |
| Free form of compound of Chemical Formula 1 | 0.75 | 1.89 | 0.01 | 6.47 | 0.07 | 5.02 |
| Hydrochloride of compound of Chemical Formula 1 | 2.19 | 2.06 | 0.02 | 3.64 | 0.14 | 4.95 |
| Sulfate of compound of Chemical Formula 1 | 8.60 | 1.83 | 0.10 | 2.86 | 0.13 | 4.91 |
| Methanesulfonate of compound of Chemical Formula 1 | 2.12 | 1.73 | 0.01 | 5.20 | 0.06 | 5.00 |
| p-Toluenesulfonate of compound of Chemical Formula 1 | 0.81 | 1.97 | 0.06 | 5.67 | 0.03 | 4.86 |
| Maleate of compound of Chemical Formula 1 | 1.10 | 2.02 | 0.01 | 4.70 | 0.19 | 4.97 |

As shown in Table 6, most of the salts exhibited higher solubility in the SGF, FaSSIF and FeSSIF than the compound of Chemical Formula 1, and among them, the sulfate exhibited about 11 times higher solubility in the SGF (pH 1.8), about 10 times higher solubility in the FaSSIF (pH 6.53), and 2 times higher solubility in the FeSSIF (pH 4.98) than the free form. Particularly, in the SGF related to the stomach in which oral formulations are first degraded in the body, the sulfate among the salt compounds of the compound of Chemical Formula 1 exhibited the highest solubility.

### Example 3-2: Physicochemical stability test

In order to assess the stability of the salt compounds of the compound of Chemical Formula 1 of the present invention, a physicochemical test was conducted under a high-temperature condition (60 °C), an accelerated condition (40 °C/75% humidity) and a light irradiation condition (1.2 million lux-hr), and a detailed description of the test is as follows.

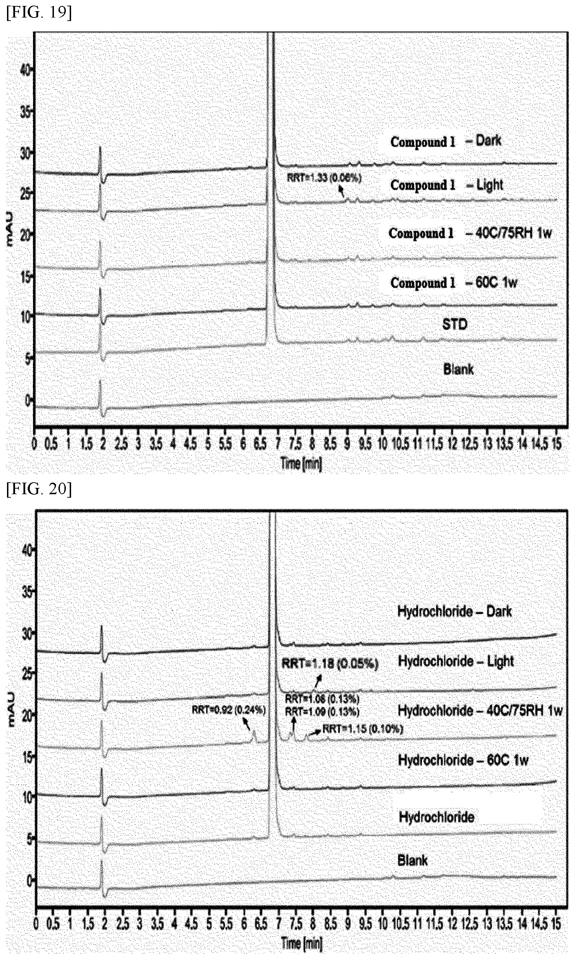

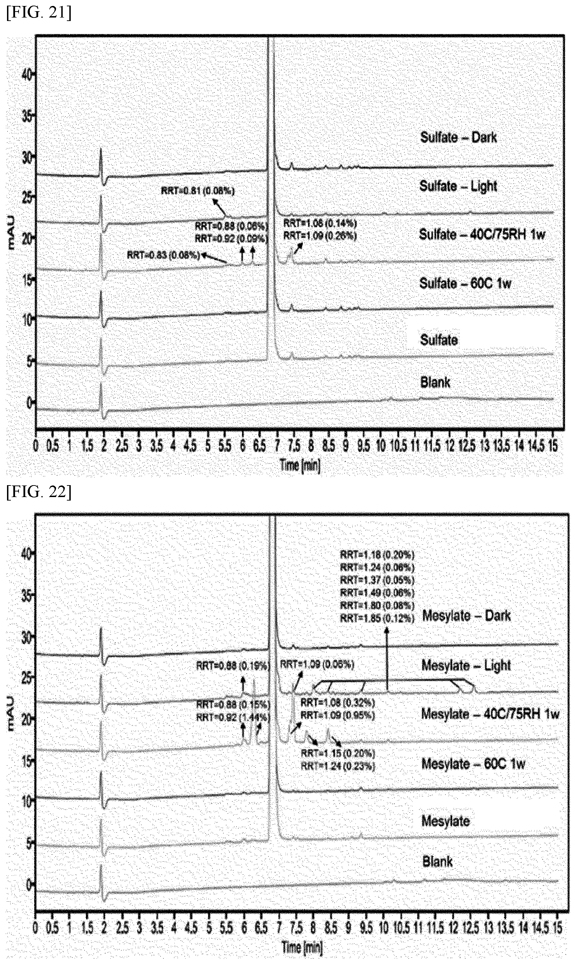

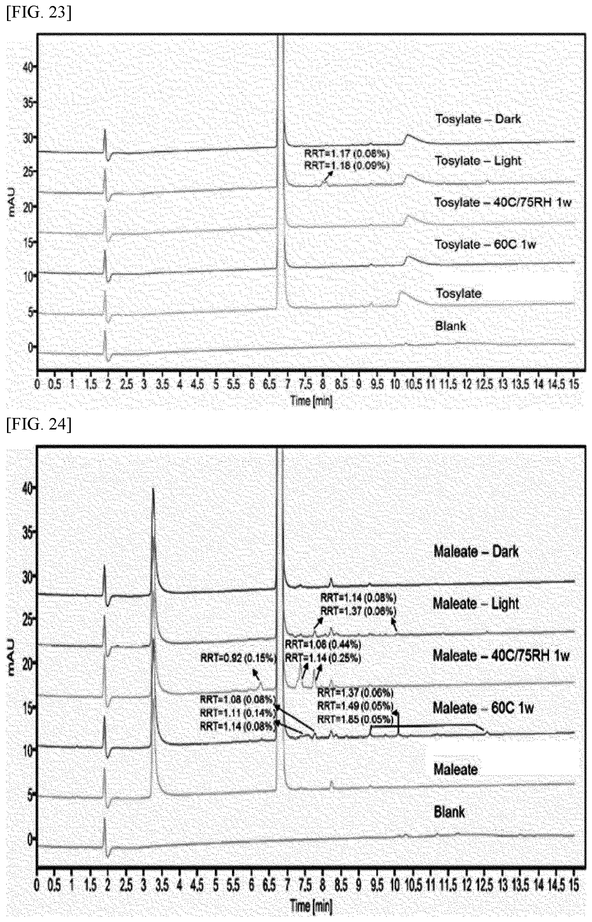

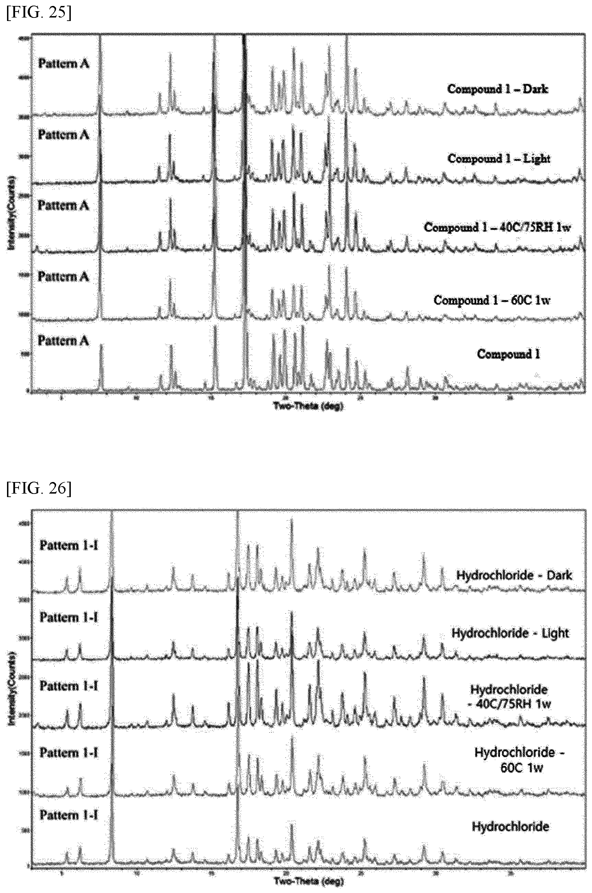

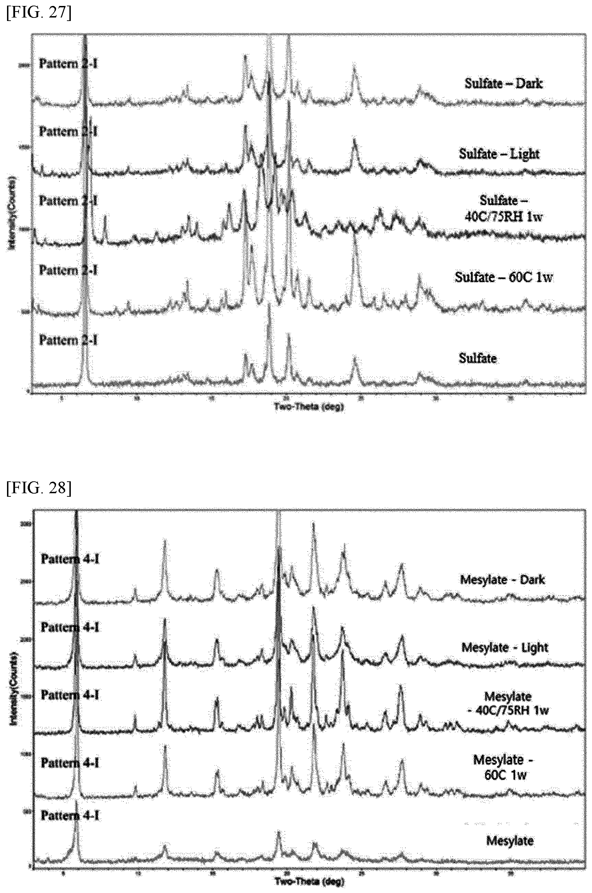

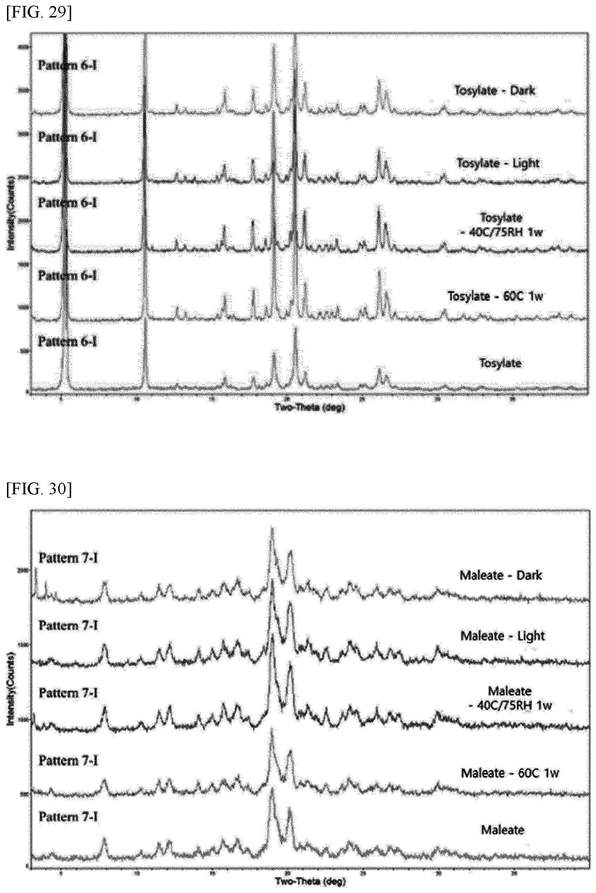

About 1 mg of the salts of the compound of Chemical Formula 1 was each input into a 2 mL vial and maintained under conditions of 60 °C and 40°C/75% humidity for 1 week. In the case of a light stability test, the vial was stored in a light stability test chamber while being not closed with a lid, and exposed to a total illuminance of 1.2 million lux-hr. A vial light-shielded with aluminum foil was used as an experimental control (Dark control). For stability assessment, state assessment by visual observation, purity assessment using HPLC, and crystalline form assessment using XRPD were conducted, and results thereof are shown in Table 7. FIGS. 19 to 24 show the HPLC spectra of the compound of Chemical Formula 1 and the 5 types of salts of the compound of Chemical Formula 1, and FIGS. 25 to 30 show the XRPD graphs of the free form of the compound of Chemical Formula 1 and the 5 types of salts of the compound of Chemical Formula 1. In FIGS. 19 to 30, "Light" represents light irradiation condition results.

**[Table 7]**

| **Stability study results for 5 types of salts of compound of Chemical Formula 1** | | | | | | |
|---|---|---|---|---|---|---|
| Starting materials | Conditions | Appearance | XRPD patterns | Final purity (%) | Purity change (%) | Assay (%) |
| Free form of compound 1 (99.76%) | 60 °C, 1 w | White powder | Pattern A | 99.94 | +0.18 | 105.89 |
| | 40 °C/75%RH, 1 w | White powder | Pattern A | 99.95 | +0.19 | 110.59 |
| | 1.2 million lux-hr | White powder | Pattern A | 99.89 | +0.13 | 107.41 |
| | Dark control | White powder | Pattern A | 99.94 | +0.18 | 109.40 |
| Hydrochloride (purity: 99.95%) | 60 °C, 1 w | White powder | Pattern 1-1 | 100.0 | +0.05 | 110.29 |
| | 40 °C/75%RH, 1 w | White powder | Pattern 1-1 | 99.40 | -0.55 | 109.18 |
| | 1.2 million lux-hr | Light yellow-brown powder | Pattern 1-1 | 99.95 | 0 | 108.65 |
| | Dark control | White powder | Pattern 1-1 | 100.0 | +0.05 | 108.99 |
| Sulfate (purity: 99.73%) | 60 °C, 1 w | White powder | Pattern 2-1 | 99.83 | +0.1 | 110.59 |
| | 40 °C/75%RH, 1 w | White powder | Pattern 2-1 | 99.30 | -0.43 | 109.86 |
| | 1.2 million lux-hr | Light yellow-brown powder | Pattern 2-1 | 99.87 | +0.14 | 104.98 |
| | Dark control | White powder | Pattern 2-1 | 99.75 | +0.02 | 105.93 |
| Mesylate (purity: 100%) | 60 °C, 1 w | White powder | Pattern 4-1 | 100.0 | 0 | 107.36 |
| | 40 °C/75%RH, 1 w | White powder | Pattern 4-1 | 96.71 | -3.29 | 101.35 |
| | 1.2 million lux-hr | Yellow-brown powder | Pattern 4-1 | 99.18 | -0.82 | 102.69 |
| | Dark control | White powder | Pattern 4-1 | 100.0 | 0 | 103.03 |
| Tosylate (purity: 99.01%) | 60 °C, 1 w | White powder | Pattern 6-1 | 98.96 | -0.05 | 108.33 |
| | 40 °C/75%RH, 1w | White powder | Pattern 6-1 | 99.00 | -0.01 | 107.73 |
| | 1.2 million lux-hr | Yellow-brown powder | Pattern 6-1 | 98.77 | -0.24 | 105.78 |
| | Dark control | White powder | Pattern 6-1 | 98.98 | -0.03 | 108.98 |
| Maleate (purity: 97.03%) | 60 °C, 1 w | Yellow powder | Pattern 7-1 | 96.59 | -0.44 | 110.00 |
| | 40 °C/75%RH, 1w | Yellow powder | Pattern 7-1 | 96.26 | -0.77 | 108.85 |
| | 1.2 million lux-hr | Yellow-brown powder | Pattern 7-1 | 96.86 | -0.17 | 109.56 |
| | Dark control | Yellow powder | Pattern 7-1 | 97.04 | +0.01 | 109.66 |

As shown in Table 7, the maleate of the compound of Chemical Formula 1 was shown to be sensitive under all conditions including heat, humidity and light irradiation conditions, and the methanesulfonate of the compound of Chemical Formula 1 was shown to be sensitive under humidity and light conditions, and particularly, a purity decrease of about 3% or more was observed at 40 °C RH for 1 week. The hydrochloride of the compound of Chemical Formula 1 and the sulfate of the compound of Chemical Formula 1 were shown to be sensitive under a humidity condition, and the p-toluenesulfonate of the compound of Chemical Formula 1 was shown to be sensitive to light. Also, all 5 types of salts showed a color change after the light stability test.

### Example 3-3: Pharmacokinetic test

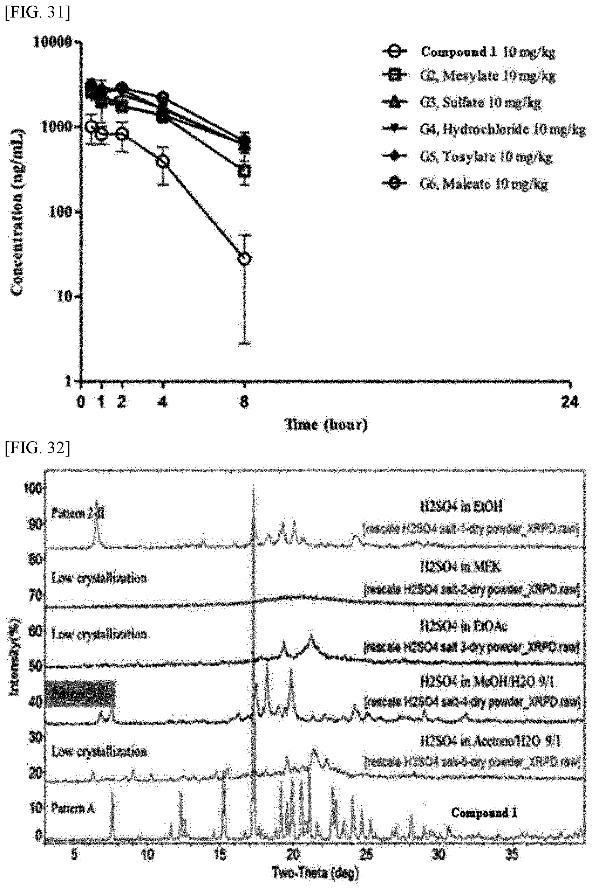

For the free form of the compound of Chemical Formula 1 and the 5 types of salt compounds of the compound of Chemical Formula 1 of the present invention, an oral administration pharmacokinetic test using mice was conducted, and results thereof are shown in Table 8 and FIG. 31.

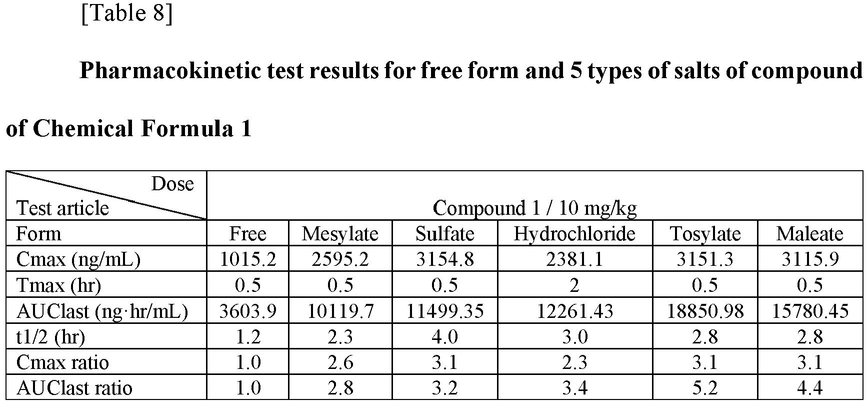

As shown in Table 8, the free form exhibited a median Tmax of 0.5 hours and was shown to be rapidly absorbed and have a half-life of 1.2 hours. The methanesulfonate, sulfate, p-toluenesulfonate, and maleate of the compound of Chemical Formula 1 exhibited a Tmax of 0.5 hours, which was equivalent to that of the free form of Compound 1, but were shown to have a longer half-life of 2.3 to 3.0 hours. The hydrochloride exhibited a Tmax of 2 hours and was shown to be relatively slowly absorbed.

In addition, as a result of comparing the degree of in vivo exposure of the free form of the compound of Chemical Formula 1 and the 5 types of salt compounds of the compound of Chemical Formula 1 of the present invention, all salts was shown to exhibit a higher degree of in vivo exposure compared to the free form, and there was no significant difference in the degree of exposure between the salts.

### Summary of Example 3

Results of the tests conducted in Examples 3-1 to 3-3 are summarized in the following Table 9.

**[Table 9]**

| **Final comparative test results for free form and 5 types of salts of compound of Chemical Formula 1** | | | | | | | | |
|---|---|---|---|---|---|---|---|---|
| | Stimulated gastrointestinal fluid solubility (mg/mL) | | | Chemical stability (purity change %/1 w) | | | Pharmacokinetic test | |
| | SG S | FaSSI F | FeSSI F | 60 ° C | 40 °C/75%R H | Light stabilit y | AUC (ng·hr/mL ) | t1/ 2 (hr) |
| Free form of compound of Chemical Formula 1 | 0.75 | 0.01 | 0.07 | 0.18 | 0.19 | 0.13 | 3603.90 | 1 |
| Hydrochloride | 2.19 | 0.02 | 0.14 | 0.18 | -0.05 | 0 | 12261.43 | 3 |
| Sulfate | 8.6 | 0.1 | 0.13 | 0.1 | -0.43 | 0.14 | 11499.35 | 4 |
| Methanesulfonat e | 2.12 | 0.01 | 0.06 | 0 | -3.29 | -0.82 | 10119.70 | 2 |
| p-Toluenesulfonate | 0.81 | 0.06 | 0.03 | -0.05 | -0.01 | -0.24 | 18850.98 | 3 |
| Maleate | 1.1 | 0.01 | 0.19 | -0.44 | -0.77 | -0.17 | 15780.45 | 3 |

As shown in Table 9, the results of the simulated gastrointestinal fluid solubility test, stability test and pharmacokinetic test show that, among the salts obtained by salt screening of Example 2, the sulfate is the best salt capable of increasing absorption into the body by improving very low solubility while maintaining excellent pharmacological properties, as compared to the free form and other salts.

More specifically, the result of the simulated gastrointestinal fluid solubility test shows that the sulfate of the compound of Chemical Formula 1 exhibited about 11 times higher solubility in the SGF (pH 1.8), about 10 times higher solubility in the FaSSIF (pH 6.53), and 2 times higher solubility in the FeSSIF (pH 4.98) than the free form of the compound of Chemical Formula 1. Also, in the stability test, the sulfate of the compound of Chemical Formula 1 was shown to exhibit comparable results to the free form of the compound of Chemical Formula 1 under high-temperature and light stability conditions, except for a humidity condition. In the pharmacokinetic comparison test using mice, when administered at the same dose as the free form of the compound of Chemical Formula 1, the sulfate of the compound of Chemical Formula 1 exhibited about 3.2 times higher absorption into the body (the free from of the compound of Chemical Formula 1: 3603.9 ng hr/mL vs the sulfate of the compound of Chemical Formula 1: 11499.35 ng·hr/mL).

Based on the above results, the sulfate of the compound of Chemical Formula 1 was selected as the final salt compound. Also, a crystalline form of the sulfate of the compound of Chemical Formula 1, which was prepared by the salt screening of Example 2, was named Crystalline form 2-I or Crystalline form I. In addition, crystalline forms (or patterns) newly found in the additional process and study on various crystalline forms of the sulfate of the compound of Chemical Formula 1 were named in the order of 2-II, 2-III, 2-IV, 2-V, 2-VI, 2-VII and 2-VIII, and they refer to Crystalline forms II, III, IV, V, VI, VII and VIII, respectively.

### Example 4: Process study for sulfate

In order to secure a convenient process that can be used for mass production of the compound of Chemical Formula 1, which was selected in Example 3, and prevent solvation, a process study was conducted. Based on the solubility results of the compound of Chemical Formula 1, ethanol, MEK, ethyl acetate, methanol/water (9:1) and acetone/water (9:1) were selected as process screening solvents for the sulfate of the compound of Chemical Formula 1. Process procedures and results using solvents are as follows.

### Example 4-1: EtOH solvent

500 mg of the compound of Chemical Formula 1 was added to 7.5 mL of ethanol, dissolved while stirring at 51 °C and 500 rpm, and maintained at 50 °C for 1 hour. To the resulting solution, 1.1 equivalents (2.502 mL, 0.5 mol/L) of a solution containing ethanol and sulfuric acid was added dropwise for 4 hours and maintained at 50 °C for 22 hours. Afterward, the resulting mixture was cooled to 5 °C for 9 hours and maintained at 5 °C for 24 hours, the suspension was filtered to collect a solid content, and the solid content was washed with ethanol and vacuum-dried at 40 °C for 16 hours to obtain 612.0 mg of a dry white solid.

### Example 4-2: MEK solvent

Example 4-2 was conducted in the same manner as in Example 4-1, except that methyl ethyl ketone (MEK) was used instead of ethanol, and a solution containing MEK and sulfuric acid was used instead of a solution containing ethanol and sulfuric acid. As a result, 518.7 mg of a dry powder (yellow solid) was obtained.

### Example 4-3: EtOAc solvent

Example 4-3 was conducted in the same manner as in Example 4-1, except that ethyl acetate was used instead of ethanol, and a solution containing ethyl acetate and sulfuric acid was used instead of a solution containing ethanol and sulfuric acid. As a result, 557.3 mg of a dry white powder (white solid) was obtained.

### Example 4-4: MeOH/H₂O (9/1) solvent

Example 4-4 was conducted in the same manner as in Example 4-1, except that methanol/water (9/1) was used instead of ethanol, and a solution containing methanol/water (9/1) and sulfuric acid was used instead of a solution containing ethanol and sulfuric acid. As a result, 587.3 mg of a dry white powder (white solid) was obtained.

### Example 4-5: Acetone/H₂O (9/1) solvent

Example 4-5 was conducted in the same manner as in Example 4-1, except that acetone/water (9/1) was used instead of ethanol, and a solution containing acetone/water (9/1) and sulfuric acid was used instead of a solution containing ethanol and sulfuric acid. As a result, 563.3 mg of a dry white powder (white solid) was obtained.

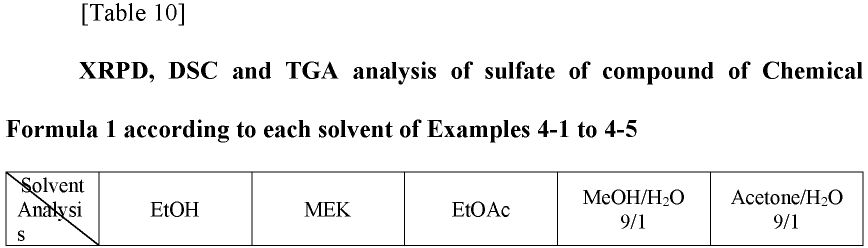

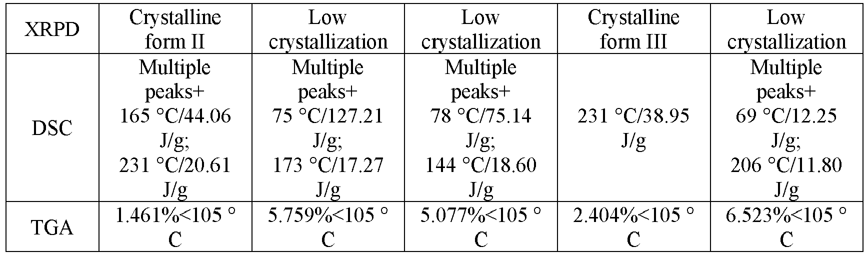

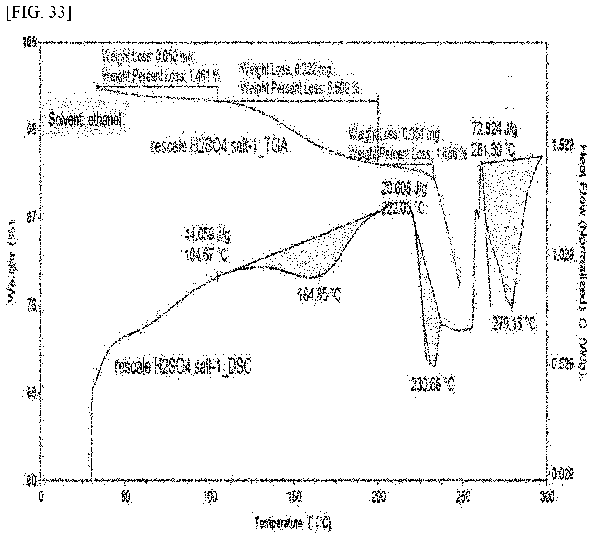

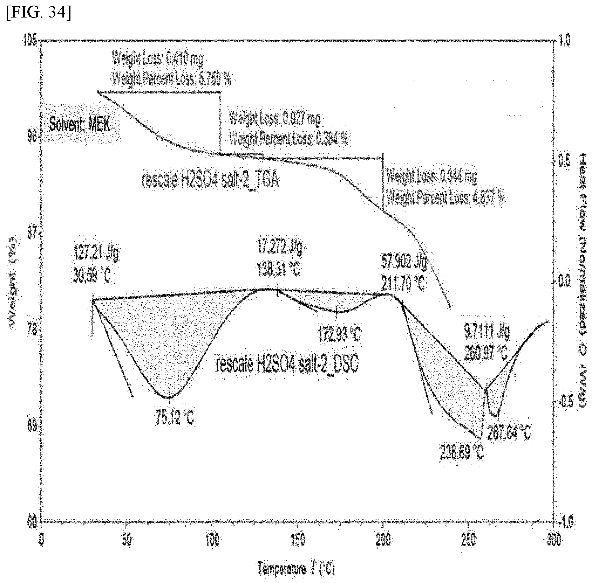

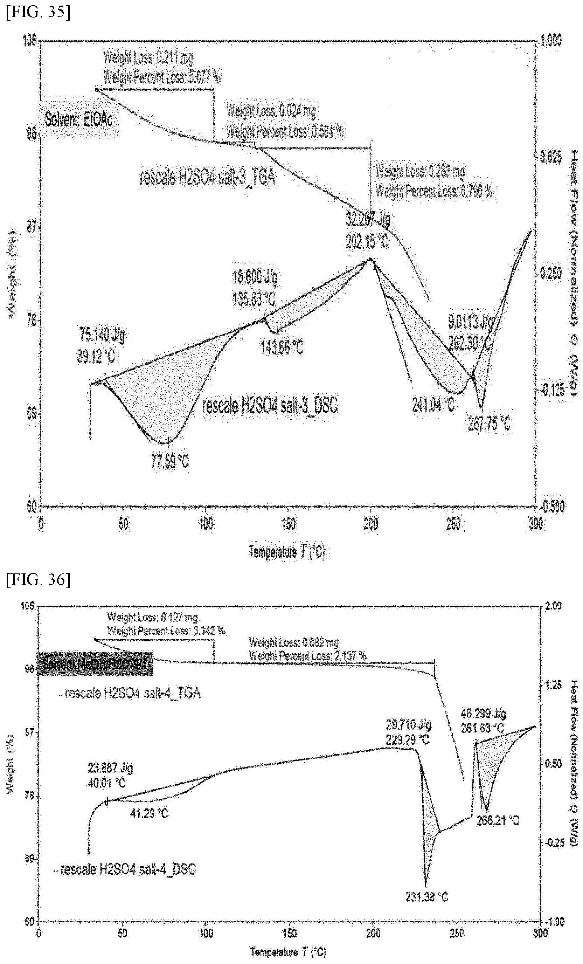

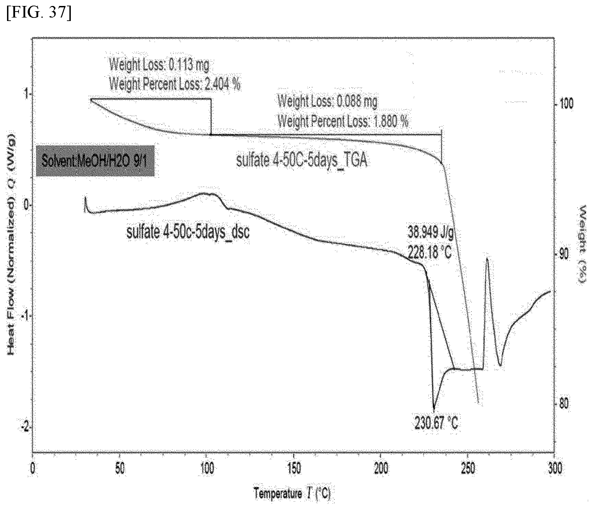

Table 10 shows the XRPD, DSC and TGA results for the sulfate of the compound of Chemical Formula 1, which was prepared using various solvents of Examples 4-1 to 4-5. FIG. 32 shows the XRPD results for the sulfate of the compound of Chemical Formula 1, which was produced under 5 types of solvents, and FIGS. 33 to 38 show the DSC/TGA results for the sulfate of the compound of Chemical Formula 1 according to respective solvents.

As a screening result of Examples 4-1 to 4-5, it was found that, when ethanol and methanol/water (9:1) were used as solvents, two types of crystalline forms were produced and named Crystalline form II and Crystalline form III, respectively.

### Example 5: Study on various crystalline forms of sulfate

In order to find a physically stable crystalline form of the sulfate of the compound of Chemical Formula 1, a study on various crystalline forms was conducted. First, solvent screening was performed for a study on various crystalline forms. More specifically, about 3 mg of the sulfate of the compound of Chemical Formula 1, which has Crystalline form III, was input into a 2 mL vial, and various solvents were added to conduct a solubility test in each solvent.

**[Table 11]**

| **Solubility results for sulfate of compound of Chemical Formula 1 in various solvents** | | | | | |
|---|---|---|---|---|---|
| Solvents | Solubility (mg/mL) | | Solvents | Solubility (mg/mL) | |
| | 25 °C | 50 °C | | 25 °C | 50 °C |
| MeOH | S<2 | 2<S<3 | THF | S<2 | S<2 |
| EtOH | S<2 | S<2 | 2-MeTHF | S<2 | S<2 |
| IPA | S<2 | S<2 | NMP | 156<S<208 | 202<S<304 |
| 1-Butanol | S<2 | S<2 | DMSO | 158<S<210 | 301<S<602 |
| ACN | S<2 | S<2 | CHCl₃ | S<2 | S<2 |
| Acetone | S<2 | S<2 | Toluene | S<2 | S<2 |
| MEK | S<2 | S<2 | Heptane | S<2 | S<2 |
| MIBK | S<2 | S<2 | Water | S<2 | S<2 |
| EtOAc | S<2 | S<2 | MeOH/H₂O=1:1(v/v) | 2<S<3 | 9<S<19 |
| iPrOAc | S<2 | S<2 | Acetone-H₂O (1:2) | 9<S<19 | 37<S<75 |
| MTBE | S<2 | S<2 | Acetic acid-H₂O (1:1) | 196<S<295 | 300<S<599 |
| DMF | 202<S<302 | 304<S<608 | - | - | - |

As shown in Table 11, the sulfate of the compound of Chemical Formula 1 exhibited an excellent solubility of 150 mg/mL or more in DMF, NMP, DMSO and acetic acid/water (1:1 (v:v)) and a good solubility of 5 to 40 mg/mL in MeOH/H₂O (1:1 (v:v)) and acetone/water (1:1 (v/v)). On the other hand, a low solubility of 5 mg/mL or less was exhibited in methanol, ethanol, isopropanol, 1-butanol, acetonitrile, acetone, MEK, MIBK, ethyl acetate, isopropyl acetate, MTBE, tetrahydrofuran, chloroform, toluene, heptane and water. Accordingly, solvents that exhibit appropriate solubility were selected, and a study on various crystalline forms of the sulfate was conducted. A detailed description of the study on various crystalline forms is as follows.

### Example 5-1: Slurry method

A suspension of the sulfate of the compound of Chemical Formula 1 was prepared for each solvent as follows. Specifically, 10 mg of the sulfate of the compound of Chemical Formula 1, which has Crystalline form III, was input into a 2 mL vial, and 1.0 mL of each solvent was added to prepare a suspension with a target concentration of 10 mg/mL (solvent: ethanol, acetone, MEK, ethyl acetate, MTBE and IPA/water (95:5 (v/v))). 300 mg of the sulfate of the compound of Chemical Formula 1, which has Crystalline form III, was input into a 2 mL vial, and 0.4 mL of a solvent was added to prepare a suspension with a target concentration of 750 mg/mL (solvent: DMSO, DMF and NMP). 50 mg of the sulfate of the compound of Chemical Formula 1, which has Crystalline form III, was input into a 2 mL vial, and 1 mL of a solvent was added to prepare a suspension with a target concentration of 50 mg/ml (solvent: acetone/water (1:2 (v/v))). 25 mg of the sulfate of the compound of Chemical Formula 1, which has Crystalline form III, was input into a 2 mL vial, and 1 mL of a solvent was added to prepare a suspension with a target concentration of 25 mg/ml (solvent: methanol/water (1:1 (v/v))). Each suspension was mixed at 25 or 50 °C for 1 or 4 weeks and subjected to centrifugation and vacuum drying at 30 °C under vacuum to prepare a solid sample, and an XRPD test was conducted. As a result of the XRPD test, novel crystalline forms were found and named Crystalline forms V, VI and VII.

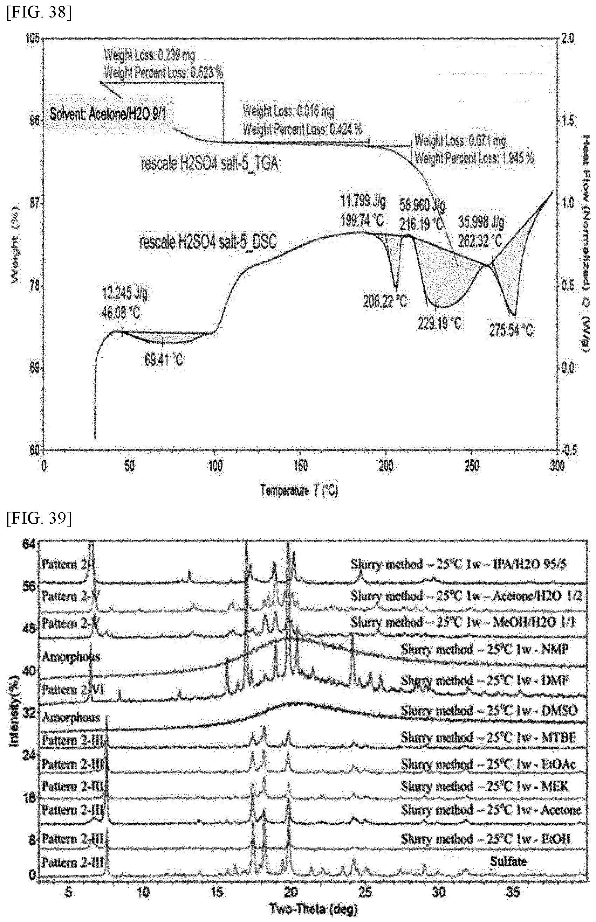

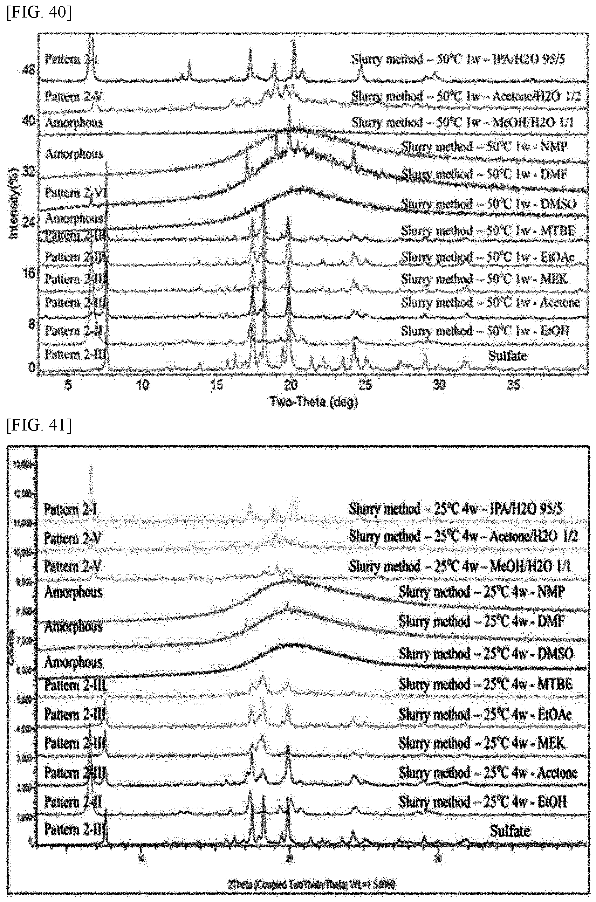

Table 12 and FIGS. 39 to 42 show the XRPD results for the sulfate of the compound of Chemical Formula 1, which was produced by slurry methods.

**[Table 12]**

| **XRPD results of sulfate of compound of Chemical Formula 1 produced by slurry methods** | | | | | | |
|---|---|---|---|---|---|---|
| Solvents | Temp. | XRPD results | | Temp. | XRPD results | |
| | | 1 week | 4 weeks | | 1 week | 4 weeks |
| EtOH | 25 °C | Pattern 2-III | Pattern 2-II | 50 °C | Pattern 2-II | Pattern 2-II |
| Acetone | | Pattern 2-III | Pattern 2-III | | Pattern 2-III | Pattern 2-III |
| MEK | | Pattern 2-III | Pattern 2-III | | Pattern 2-III | Pattern 2-III |
| EtOAc | | Pattern 2-III | Pattern 2-III | | Pattern 2-III | Pattern 2-III |
| MTBE | | Pattern 2-III | Pattern 2-III | | Pattern 2-III | Pattern 2-III |
| DMSO | | Amorphous | Amorphous | | Amorphous | Amorphous |
| DMF | | Pattern 2-VI | Amorphous | | Pattern 2-VI | Amorphous |
| NMP | | Amorphous | Amorphous | | Amorphous | Amorphous |
| MeOH/water (1:1) | | Pattern 2-V | Pattern 2-V | | Amorphous | Amorphous |
| Acetone/water (1:2) | | Pattern 2-V | Pattern 2-V | | Pattern 2-V | Pattern 2-VII |
| IPA/water (95:5) | | Pattern 2-1 | Pattern 2-1 | | Pattern 2-1 | Pattern 2-1 |

### Example 5-2: Slow evaporation method

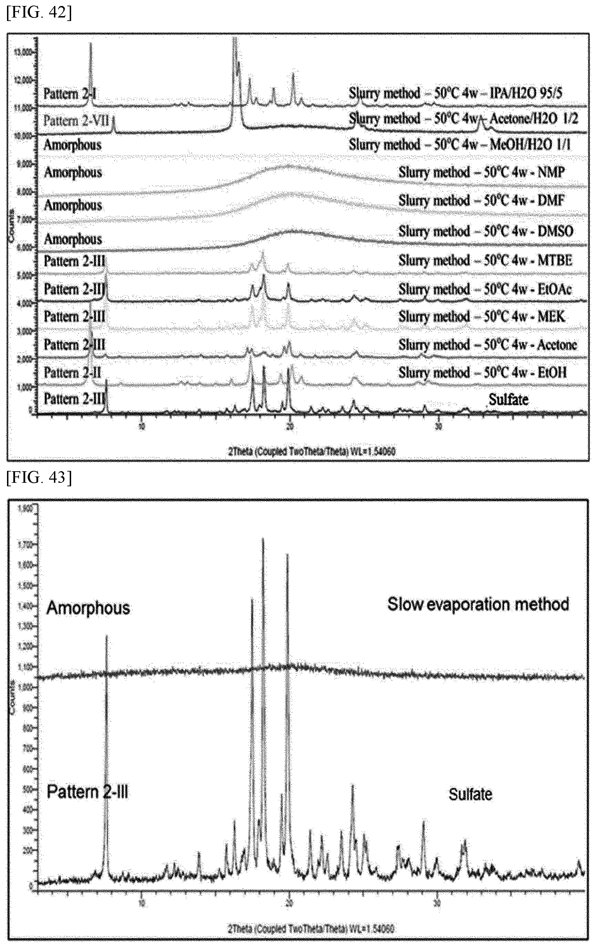

10 mg of the sulfate of the compound of Chemical Formula 1, which has Crystalline form III, was input into a 2 mL vial, acetone/water (1/2 (v/v)) was added and mixed well until completely dissolved, and then Parafilm was used as a lid. The Parafilm was pierced, and then the solvent was allowed to evaporate slowly at room temperature. After solid formation was confirmed, vacuum drying was additionally performed at 30 °C, and an XRPD test was conducted. As a result of the XRPD test, it was found that the crystalline form produced by the slow evaporation method was amorphous. FIG. 43 shows an XRPD result of the sulfate of the compound of Chemical Formula 1, which was produced by a slow evaporation method.

### Example 5-3: Heat-cooling method

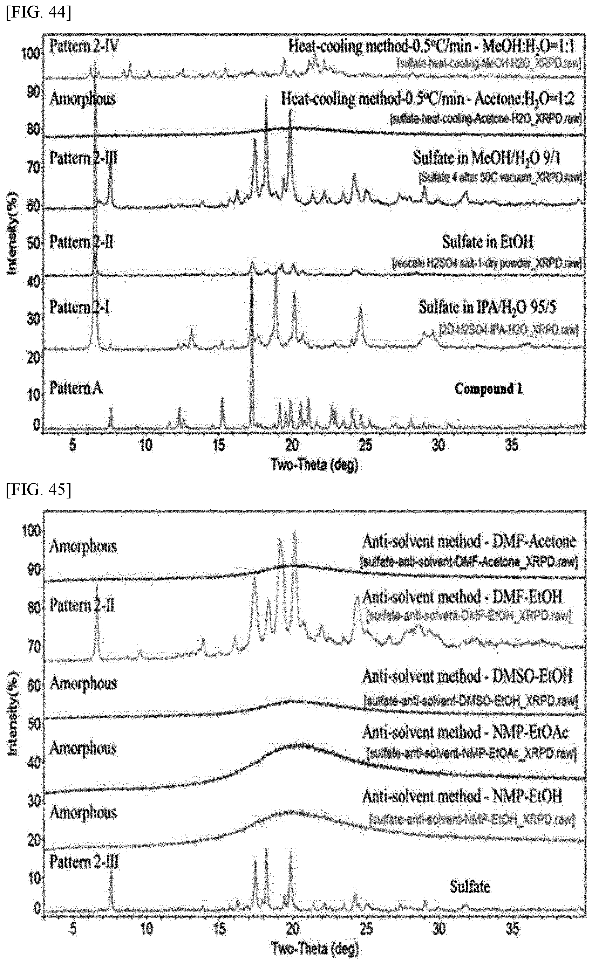

10 mg of the sulfate of the compound of Chemical Formula 1, which has Crystalline form III, was input into a 2 mL vial, methanol/water (1:1(v/v)) was added, and 25 mg of the sulfate of the compound of Chemical Formula 1, which has Crystalline form III, was input into a 2 mL vial, and acetone/water (1:2 (v/v)) was added. Each vial was subjected to complete dissolution at 50 °C, cooled from 50 °C to 0 °C at 0.5 °C/min, and maintained at 0 °C for 20 hours. The suspension was centrifuged at 14000 rpm for 5 minutes to obtain a solid, and the obtained solid was vacuum-dried at 30 °C and subjected to an XRPD test. As a result of the XRPD test, when acetone/water (1:2 (v/v)) was used as a solvent, an amorphous sulfate of the compound of Chemical Formula 1 was produced. On the other hand, when methanol/water (1:1 (v/v)) was used, a novel crystalline form was produced and named Crystalline form IV. FIG. 44 shows an XRPD result of the sulfate of the compound of Chemical Formula 1 according to a heat-cooling method.

### Example 5-4: Anti-solvent method

About 100 mg of the sulfate of the compound of Chemical Formula 1, which has Crystalline form III, was input into each of several 4 mL vials, and DMF, NMP and DMSO were added until complete dissolution. Each vial was centrifuged, the supernatant was transferred to two new vials, and an anti-solvent was added to precipitate a solid (used solvent: anti-solvent; NMP:EtOH=1v:3v, NMP:EtOAc=1v:4v, DMSO:EtOH=1v:5v, DMSO:MTBE=1v:7v, DMF:EtOH=1v:2v and DMF:Acetone=1v:3v). The precipitated solid was centrifuged and subjected to an XRPD test. As a result of the XRPD test, it was found that Crystalline form II was produced under a DMF-ethanol solvent/anti-solvent condition. Table 13 and FIG. 45 show an XRPD result of the sulfate of the compound of Chemical Formula 1 according to an anti-solvent method.

**[Table 13]**

| **Crystalline form of sulfate of compound of Chemical Formula 1 using anti-solvent method** | | | |
|---|---|---|---|
| Solvent Name | Anti-solvents | Observation | XRPD results |
| NMP | EtOH | Suspension | Amorphous |
| | EtOAc | Suspension | Amorphous |
| DMSO | EtOH | Suspension | Amorphous |
| | MTBE | Clear | N/A |
| DMF | EtOH | Suspension | Pattern 2-II |
| | Acetone | Suspension | Amorphous |

### Example 5-5: Vapor diffusion method

10 mg of the sulfate of the compound of Chemical Formula 1 was input into a 4 mL vial, and acetone/water (1:2 (v/v)) was added until complete dissolution. The solution contained in the 4 mL vial was transferred to a 40 mL vial, 5 mL of MEK and methanol were added as an anti-solvent, and the vial was closed with a lid and stored for 4 weeks. As a result of the XRPD test, no novel crystalline form was produced.

### Example 5-6: Crystallization method using melting-cooling method

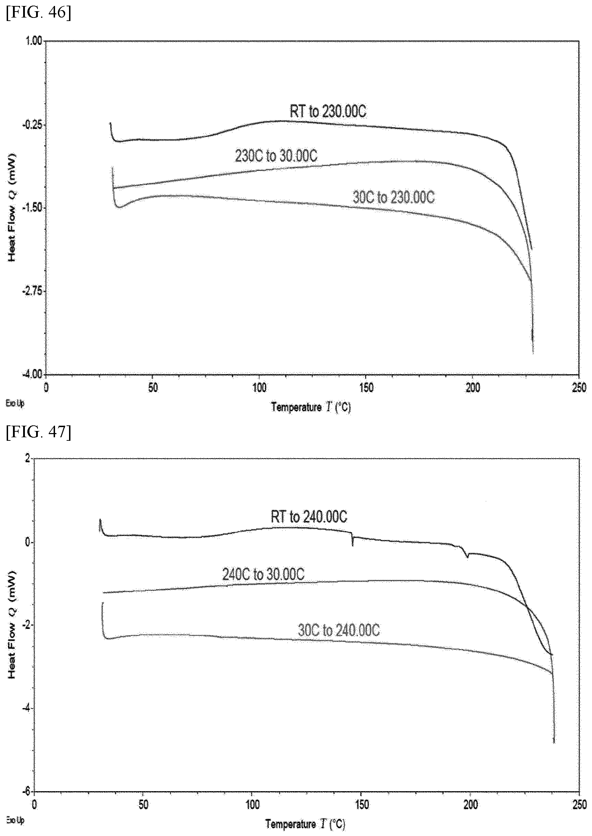

Crystallization method 1: 3 mg of the sulfate of the compound of Chemical Formula 1, which has Crystalline form III, was input into an airtight aluminum pan, heated from room temperature to 230 °C at 10 °C/min, cooled from 230 °C to 30 °C at 10 °C/min, and heated again from 30 °C to 275 °C at 10 °C/min. FIG. 46 shows a DSC result of the sulfate of the compound of Chemical Formula 1, which was produced by crystallization method 1.

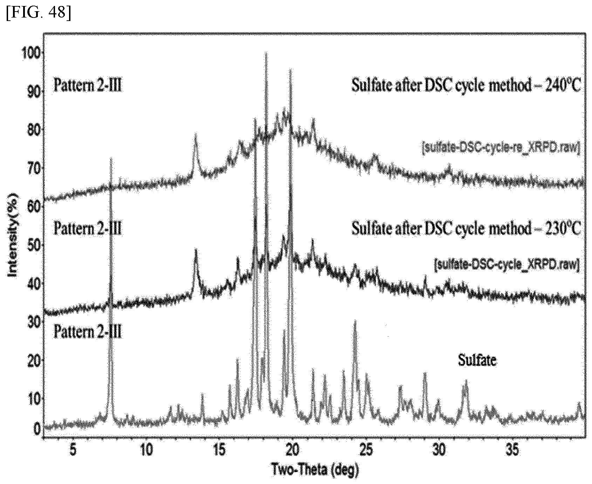

Crystallization method 2: 3 mg of the sulfate of the compound of Chemical Formula 1, which has Crystalline form III, was input into an airtight aluminum pan, heated from room temperature to 240 °C at 10 °C/min, cooled from 240 °C to 30 °C at 10 °C/min, and heated again from 30 °C to 275 °C at 10 °C/min. According to an XRPD result, no novel crystalline form was produced. FIG. 47 shows a DSC result of the sulfate of the compound of Chemical Formula 1, which was produced by crystallization method 2. FIG. 48 shows an XRPD result of the sulfate of the compound of Chemical Formula 1, which was produced by crystallization methods 1 and 2.

### Example 5-7: Water sorption and desorption method (dynamic vapor sorption (DVS) cycle)

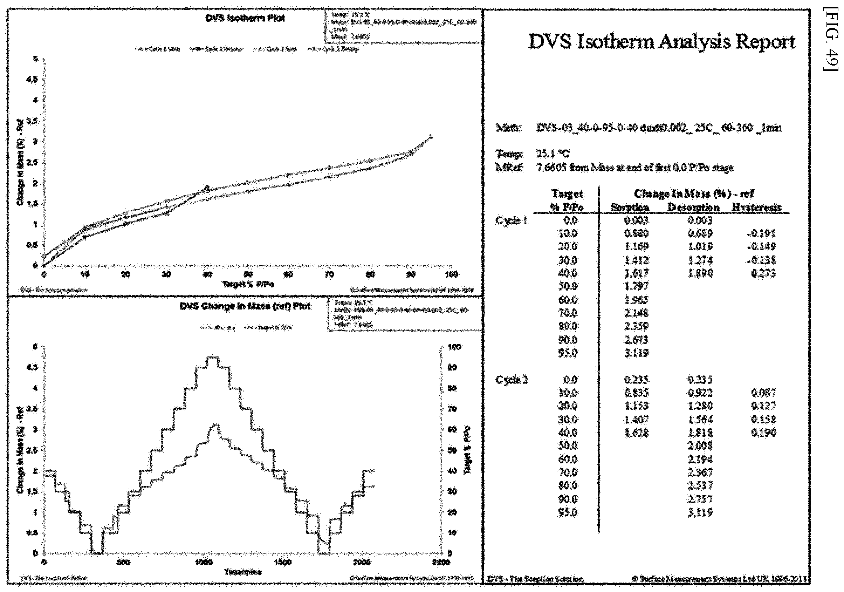

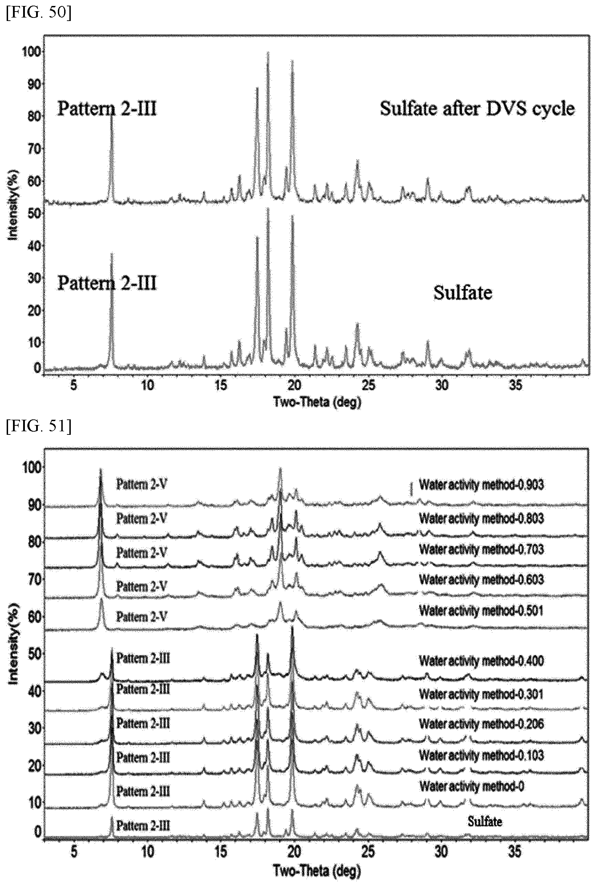

8 mg of the sulfate of the compound of Chemical Formula 1, which has Crystalline form III, was placed in a DVS instrument (SMS DVS INTRINSIC system), a mass change in a room temperature/atmospheric state was recorded, and an XRPD test was conducted. As an XRPD result, no novel crystalline form was produced. FIG. 49 shows a DVS result of the sulfate of the compound of Chemical Formula 1, and FIG. 50 shows an XRPD result of the sulfate of the compound of Chemical Formula 1 after a DVS test.

### Example 5-8: Water activity test

50 mg of the sulfate of the compound of Chemical Formula 1, which has Crystalline form III, was input into several 2 mL vials, and 1 mL of each water activity solution (0, 0.103, 0.206, 0.301, 0.400, 0.501, 0.603, 0.703, 0.803 and 0.901) was added to produce a suspension with a target concentration of 50 mg/mL. Each vial was closed with a lid and stirred at 25 °C and 800 rpm for 3 days. The sample was centrifuged, vacuum-dried at 30 °C, and subjected to an XRPD test. According to an XRPD result, although a novel crystalline form was produced, it was the same crystalline form as the above-described Crystalline forms III and V. Table 14 and FIG. 51 show an XRPD result of the sulfate of the compound of Chemical Formula 1 according to a water activity test.

**[Table 14]**

| **Water activity test of sulfate of compound of Chemical Formula 1** | | | | |
|---|---|---|---|---|
| No. | Water activity | Water/acetone (%, v/v) | Observation | XRPD results |
| 1 | 0 | 0 | suspension | Pattern 2-III |
| 2 | 0.103 | 0.6% | suspension | Pattern 2-III |
| 3 | 0.206 | 1.5% | suspension | Pattern 2-III |
| 4 | 0.301 | 2.8% | suspension | Pattern 2-III |
| 5 | 0.400 | 5.0% | suspension | Pattern 2-III |
| 6 | 0.501 | 8.6% | suspension | Pattern 2-V |
| 7 | 0.603 | 14.5% | suspension | Pattern 2-V |
| 8 | 0.703 | 24.0% | suspension | Pattern 2-V |
| 9 | 0.803 | 40.0% | suspension | Pattern 2-V |
| 10 | 0.901 | 65.0% | suspension | Pattern 2-V |

### Example 5-9: Compressive behavior test

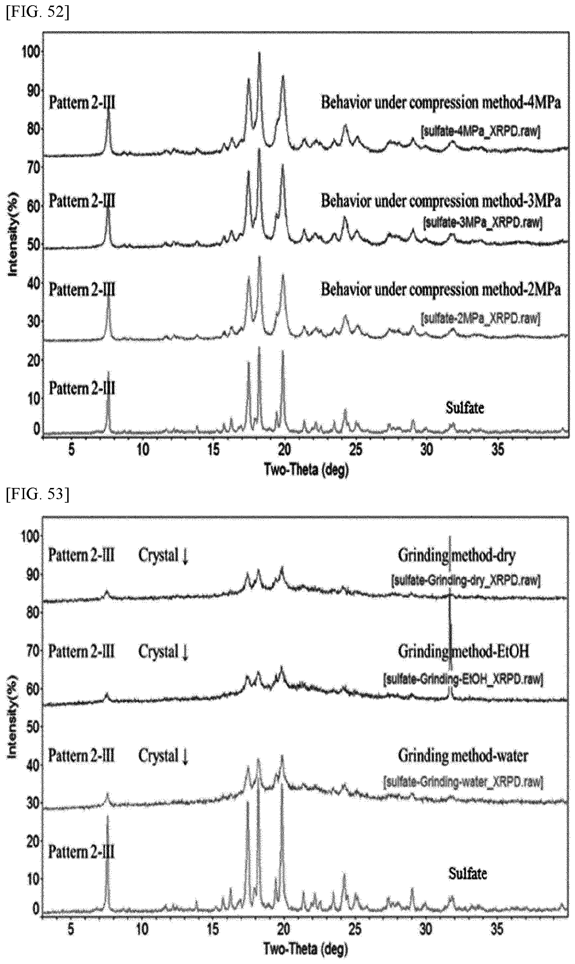

100 mg of the sulfate of the compound of Chemical Formula 1, which has Crystalline form III, was input into a 2 mL vial, the vial was transferred to a die cavity, an upper punch was mounted on the die cavity, and pressures of 2, 3 and 4 MPa was applied for 3 minutes to produce compressed pellets. As a result of the XRPD test, no novel crystalline form was produced. FIG. 52 shows an XRPD result of the sulfate of the compound of Chemical Formula 1 according to a compressive behavior test.

### Example 5-10: Grinding method

10 mg of the sulfate of the compound of Chemical Formula 1, which has Crystalline form III, was ground using a mortar for 5 minutes (dry grinding method). 10 mg of the sulfate of the compound of Chemical Formula 1, which has Crystalline form III, was ground with 20 uL of ethanol using a mortar for 5 minutes (wet grinding method using ethanol). 10 mg of the sulfate of the compound of Chemical Formula 1, which has Crystalline form III, was ground with 20 uL of water using a mortar for 5 minutes (wet grinding method using water). As a result of the XRPD test, no novel pattern was produced. FIG. 53 shows an XRPD result of the sulfate of the compound of Chemical Formula 1 according to a grinding method.

### Summary of Example 5

A total of 4 novel crystalline forms were found through the study on various crystalline forms of Example 5 in addition to Crystalline forms I to III found in Examples 3 and 4. Novel Crystalline form IV was produced by a heat-cooling method in which heat-cooling proceeded at a temperature of 50 °C and a rate of 0.5 °C in MeOH/water (1:1), and 3 novel Crystalline forms V, VI and VII were produced by a slurry method in MeOH/water (1:1), DMF and acetone/water (1:2) solvents for 1 to 4 weeks. Crystalline form V was a crystalline form also found in a system with water activity > 0.501 in the water activity test. No novel XRPD pattern was not found in the slow evaporation method, anti-solvent method, vapor diffusion method, crystallization method using a melting-cooling method, water sorption and desorption method, compressive behavior method, grinding method and the like.

In addition, Crystalline form III of the sulfate of the compound of Chemical Formula 1 was shown to be maintained under the test conditions of the melting-cooling method, water sorption and desorption method, compressive behavior method and grinding method, and thus it was confirmed it was stable under each test condition.

A total of 7 crystalline forms of the sulfate of the compound of Chemical Formula 1 were confirmed by the salt screening of Example 3, the process study of Example 4 and the study on various crystalline forms of Example 5, and as described in the summary of Example 3, crystalline forms (or patterns) were named in the order of 2-I, 2-II, 2-III, 2-IV, 2-V, 2-VI and 2-VII, and they refer to Crystalline forms I, II, III, IV, V, VI and VII, respectively. These crystalline forms were analyzed in Example 6 below.

### Example 6: Identification of crystalline forms of sulfate of compound of Chemical Formula 1

### Example 6-1: Identification of Crystalline form I

Crystalline form I was produced in the salt screening of the compound of Chemical Formula 1, which was performed in Example 2-2, and the IPA/water (95/5) slurry method at 25 °C and 50 °C, which was performed in Example 5-1.

**[Table 15]**

| **List of characteristic XRPD peaks of Crystalline form I of sulfate of compound of Chemical Formula 1** | | | |
|---|---|---|---|
| **Angle** | **d value** | **Intensity** | **Intensity %** |
| **2-Theta °** | **Angstrom** | **Count** | **%** |
| 6.625 | 13.331 | 2527 | 100.0 |
| 9.497 | 9.305 | 178 | 4.8 |
| 12.293 | 7.194 | 164 | 3.6 |
| 12.758 | 6.933 | 163 | 3.3 |
| 13.205 | 6.699 | 251 | 6.8 |
| 13.435 | 6.585 | 284 | 8.1 |
| 14.816 | 5.974 | 179 | 4.0 |
| 15.734 | 5.628 | 175 | 3.5 |
| 16.026 | 5.526 | 237 | 5.8 |
| 17.342 | 5.109 | 1198 | 43.7 |
| 17.757 | 4.991 | 811 | 27.8 |
| 18.698 | 4.742 | 542 | 16.5 |
| 18.933 | 4.683 | 2517 | 96.3 |
| 19.837 | 4.472 | 310 | 7.1 |
| 20.242 | 4.384 | 1794 | 67.3 |
| 20.827 | 4.262 | 485 | 14.7 |
| 21.564 | 4.118 | 395 | 11.5 |
| 22.416 | 3.963 | 167 | 2.7 |
| 24.663 | 3.607 | 817 | 28.7 |
| 25.891 | 3.438 | 228 | 5.1 |
| 26.565 | 3.353 | 232 | 5.2 |
| 27.181 | 3.278 | 190 | 3.4 |
| 28.044 | 3.179 | 229 | 4.9 |
| 29.004 | 3.076 | 405 | 12.0 |
| 33.130 | 2.702 | 198 | 3.9 |
| 36.068 | 2.488 | 221 | 4.7 |

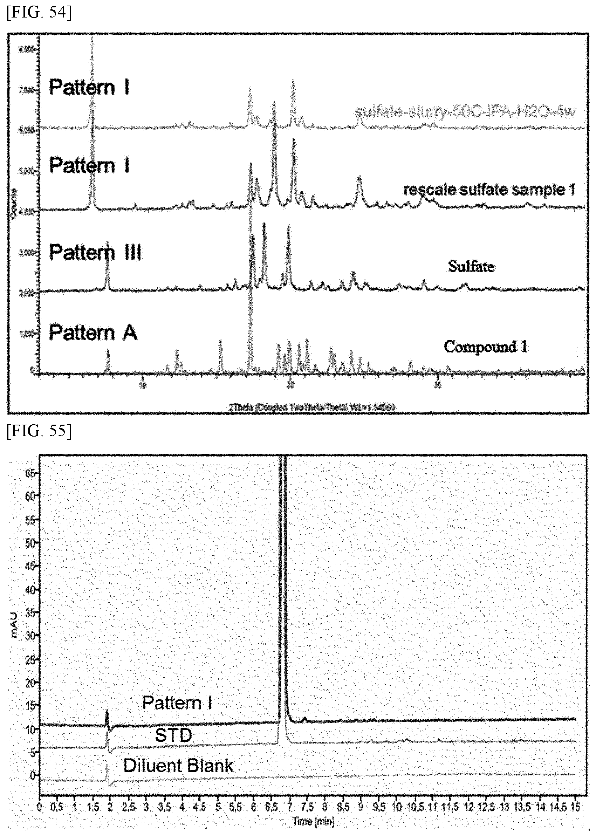

FIG. 54 and Table 15 show an XRPD result of Crystalline form I of the sulfate. Crystalline form I is identified with an X-ray powder diffraction pattern having at least 4 diffraction peaks at 2[θ] values selected from among 6.63±0.2, 17.34±0.2, 17.76±0.2, 18.70±0.2, 18.93±0.2, 20.24±0.2, 20.83±0.2, 21.56±0.2, 24.66±0.2 and 29.00±0.2. The XRPD analysis result showed that Crystalline form I was different from Crystalline form III and exhibited a purity of 99.73% as shown in the HPLC test result of FIG. 55 and 0.27% degradation occurred (RT=6.799 min).

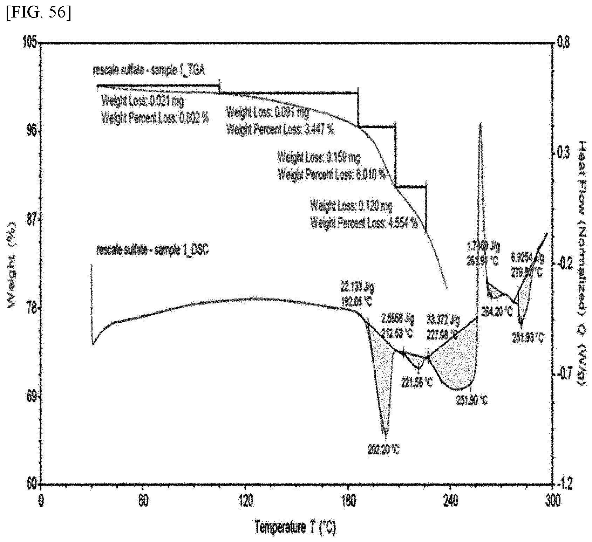

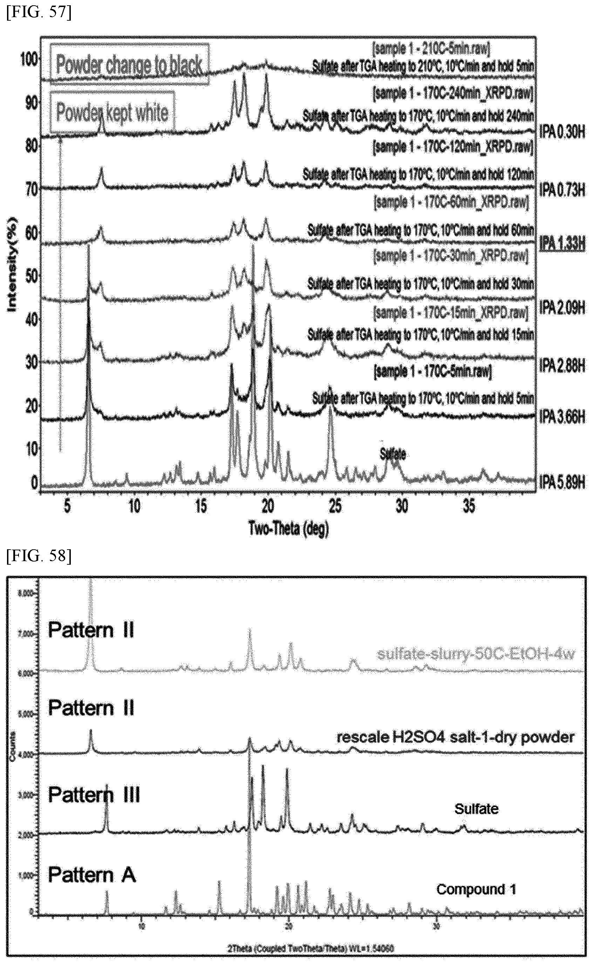

FIG. 56 shows a TGA/DSC graph of Crystalline form I, and FIG. 57 shows an XRPD result of Crystalline form I before and after TGA.

To identify a sulfate, TGA heating was performed at 170 °C and 210 °C. The DSC graph of FIG. 56 showed that TGA loss occurred at about 200 °C, which demonstrated that Crystalline form I was solvated with isopropanol (IPA). As shown in FIG. 57, even after Crystalline form I was subjected to TGA heating at 170 °C for 5 minutes, isopropanol remained (3.66H¹H NMR), and when Crystalline form I was heated at 170 °C for 60 minutes, a molar ratio of residual isopropanol The compound of Chemical Formula 1 was 2:9, and in this case, XRPD was changed, which means that Crystalline form I is an IPA solvate and removal of the IPA solvent is difficult due to high lattice energy. When heating was maintained at 170 °C for 240 minutes, IPA could be completely removed, and when heating was performed at 210 °C for 5 minutes, it turned black and degraded.

### Example 6-2: Identification of Crystalline form II

Crystalline form II was produced in the ethanol slurry method of Example 5-1, the DMF-EtOH solvent/anti-solvent method of Example 5-4 and the process study using the ethanol solvent of Example 4-1.

**[Table 16]**

| **List of characteristic XRPD peaks of Crystalline form II of sulfate** | | | |
|---|---|---|---|
| 2-Theta | d(A) | Counts | Intensity % |
| 6.569 | 13.445 | 608 | 100.0 |
| 13.908 | 6.363 | 133 | 16.2 |
| 16.054 | 5.516 | 113 | 12.3 |
| 17.362 | 5.104 | 415 | 62.1 |
| 18.405 | 4.817 | 194 | 22.6 |
| 19.156 | 4.630 | 243 | 30.7 |
| 19.358 | 4.582 | 347 | 48.8 |
| 20.109 | 4.412 | 347 | 49.0 |
| 20.769 | 4.273 | 157 | 16.9 |
| 24.311 | 3.658 | 169 | 20.7 |

FIG. 58 and Table 16 show an XRPD result of Crystalline form II of the sulfate. The Crystalline form II is identified with an X-ray powder diffraction pattern having at least 4 diffraction peaks at 2[θ] values selected from among 6.57±0.2, 13.91±0.2, 16.05±0.2, 17.36±0.2, 18.41±0.2, 19.16±0.2, 19.36±0.2, 20.11±0.2, 20.77±0.2 and 24.31±0.2.

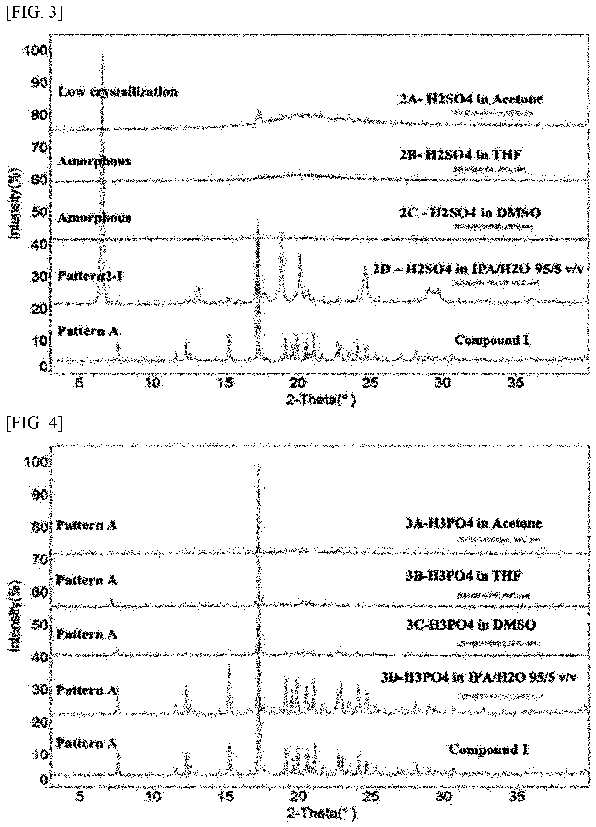

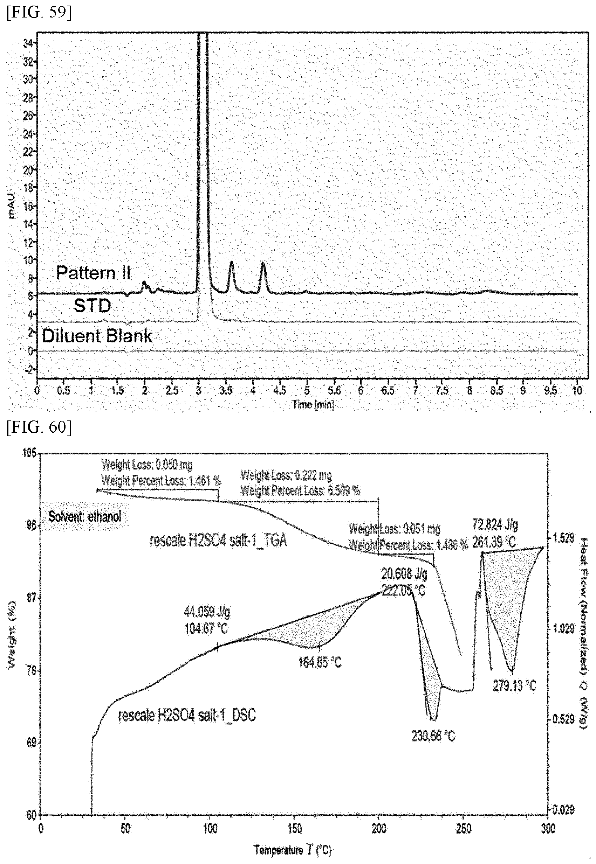

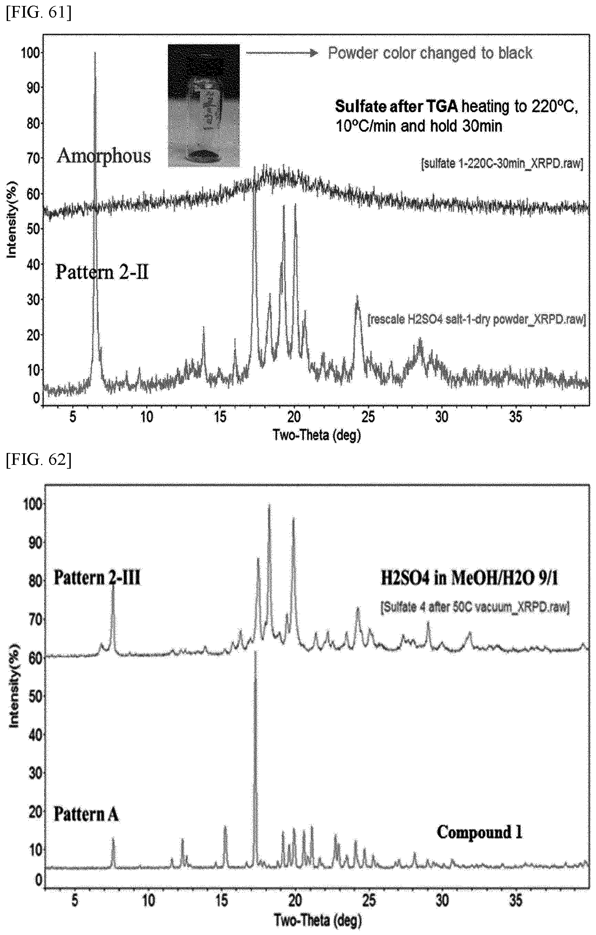

The XRPD analysis result showed that Crystalline form II was different from Crystalline form III and exhibited a purity of 96.81% as shown in the HPLC test result of FIG. 59, and 3.19% degradation occurred (RT=3.046 min). The DSC graph of FIG. 60 showed a TGA loss of 6.5% at 100 to 220 °C. As shown in FIG. 61, after being heated at 220 °C for 30 minutes, crystalline form 2-II was changed to an amorphous state, which indicates solvation.

### Example 6-3: Identification of Crystalline form III

Crystalline form III was produced in the process study using the methanol/water (9/1) solvent of Example 4-4 and the water activity test of Example 5-8.

**[Table 17]**

| **List of characteristic XRPD peaks of Crystalline form III of sulfate** | | | |
|---|---|---|---|
| 2-Theta | d(A) | Counts | Intensity % |
| 6.844 | 12.905 | 289 | 7.7 |
| 7.657 | 11.536 | 1491 | 49.7 |
| 12.255 | 7.217 | 168 | 2.5 |
| 13.913 | 6.360 | 234 | 4.6 |
| 15.275 | 5.796 | 185 | 2.7 |
| 15.786 | 5.609 | 328 | 7.1 |
| 16.318 | 5.428 | 568 | 14.8 |
| 17.532 | 5.055 | 1971 | 62.8 |
| 18.048 | 4.911 | 711 | 18.5 |
| 18.277 | 4.850 | 3044 | 100.0 |
| 18.946 | 4.680 | 521 | 11.6 |
| 19.492 | 4.550 | 877 | 24.1 |
| 19.912 | 4.455 | 2784 | 90.9 |
| 21.435 | 4.142 | 500 | 11.2 |
| 22.236 | 3.995 | 535 | 12.1 |
| 22.603 | 3.931 | 334 | 5.1 |
| 23.522 | 3.779 | 515 | 11.2 |
| 24.288 | 3.662 | 1011 | 28.5 |
| 25.102 | 3.545 | 581 | 13.6 |
| 27.374 | 3.255 | 477 | 10.5 |
| 28.054 | 3.178 | 370 | 6.6 |
| 29.070 | 3.069 | 730 | 19.4 |
| 29.990 | 2.977 | 317 | 5.4 |
| 31.904 | 2.803 | 529 | 12.8 |
| 39.624 | 2.273 | 302 | 4.0 |

FIG. 62 and Table 17 show an XRPD result of Crystalline form III of the sulfate. Crystalline form III is identified with an X-ray powder diffraction pattern having at least 4 diffraction peaks at 2[θ] values selected from among 7.66±0.2, 16.32±0.2, 17.53±0.2, 18.05±0.2, 18.28±0.2, 18.95±0.2, 19.49±0.2, 19.91±0.2, 21.44±0.2, 22.24±0.2, 23.52±0.2, 24.29±0.2, 25.10±0.2, 27.37±0.2, 29.07±0.2 and 31.90±0.2.

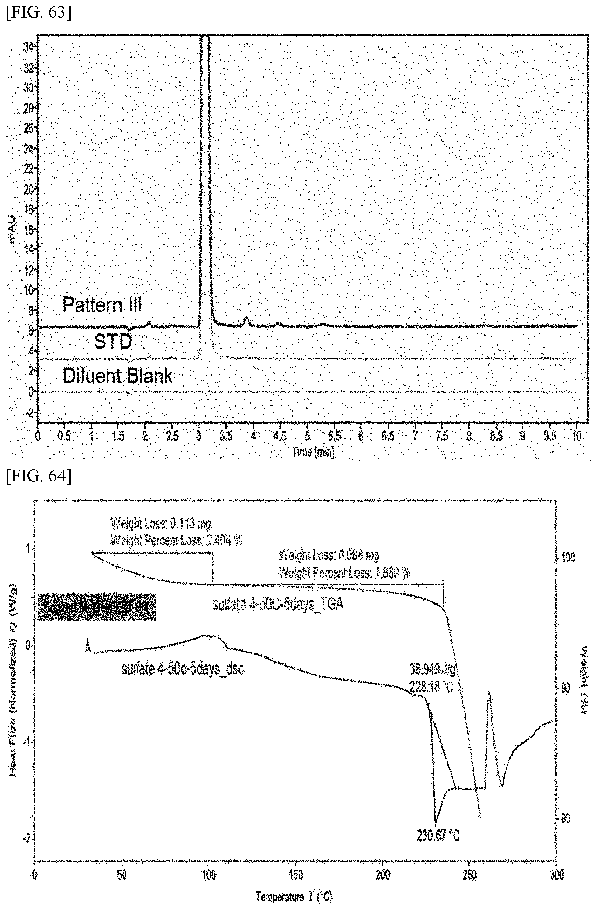

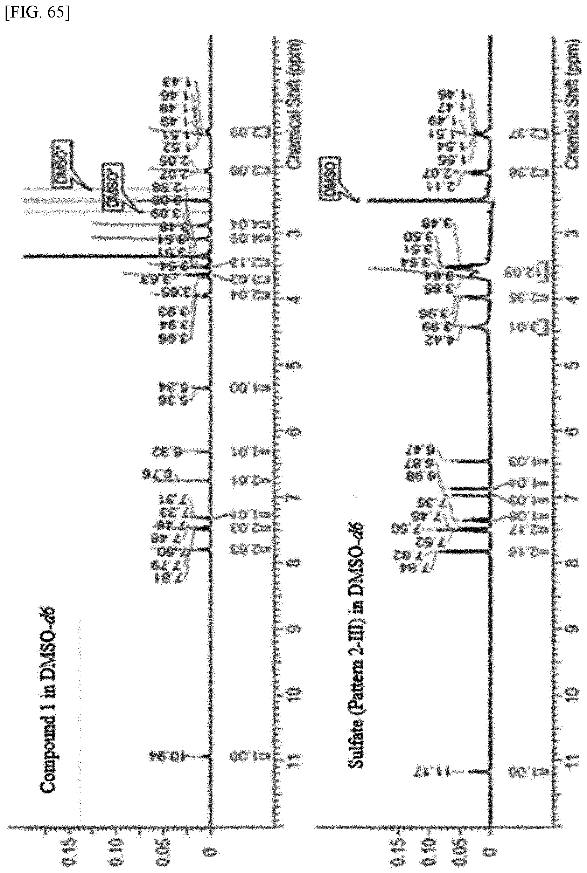

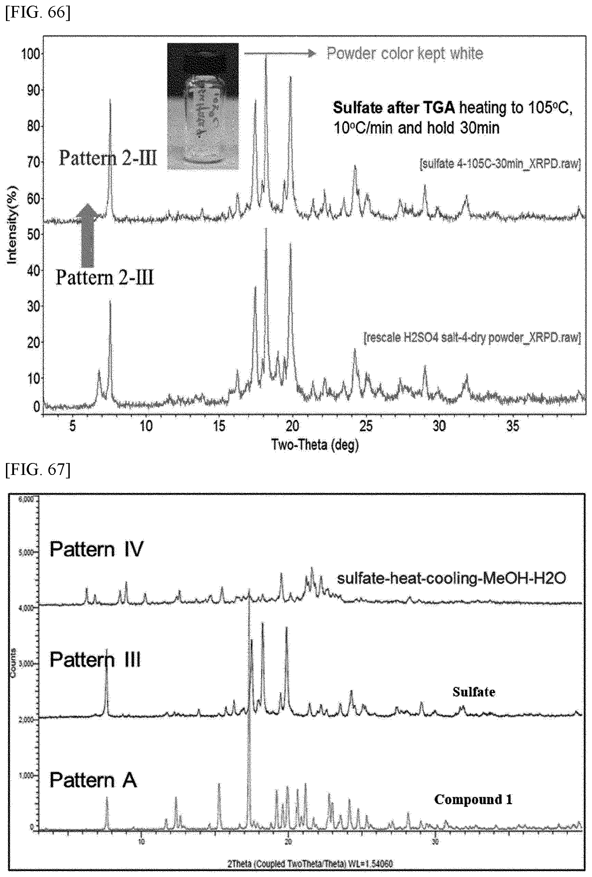

The HPLC result of FIG. 63 showed a purity of 98.41% and 1.59% degradation (RT=3.061 min). The DSC graph of FIG. 64 showed a TGA loss of 1.88% at about 230 °C. As shown in FIGS. 65 and 66, Crystalline form III did not exhibit any water or methanol peak before heating and after heating at 105 °C for 30 minutes, which indicates that pattern III is a pure sulfate crystalline form, not water or a solvate.

### Example 6-4: Identification of Crystalline form IV

Crystalline form IV was produced in the heat-cooling method (methanol/water=1:1 (v/v)) of Example 5-3.

**[Table 18]**

| **List of characteristic XRPD peaks of Crystalline form IV of sulfate** | | | |
|---|---|---|---|
| 2-Theta | d(A) | Counts | Intensity % |
| 6.296 | 14.028 | 154 | 40.3 |
| 6.870 | 12.857 | 107 | 20.1 |
| 8.576 | 10.302 | 155 | 39.9 |
| 9.020 | 9.796 | 193 | 55.4 |
| 10.262 | 8.613 | 109 | 16.8 |
| 12.457 | 7.100 | 140 | 27.1 |
| 14.711 | 6.017 | 141 | 18.9 |
| 15.504 | 5.711 | 167 | 29.1 |
| 17.416 | 5.088 | 182 | 28.7 |
| 18.269 | 4.852 | 163 | 18.0 |
| 19.558 | 4.535 | 302 | 75.7 |
| 20.200 | 4.393 | 167 | 17.0 |
| 21.554 | 4.120 | 361 | 100.0 |
| 22.284 | 3.986 | 309 | 78.6 |
| 28.309 | 3.150 | 130 | 17.3 |

FIG. 67 and Table 18 show an XRPD result of Crystalline form IV of the sulfate. Crystalline form IV is identified with an X-ray powder diffraction pattern having at least 4 diffraction peaks at 2[θ] values selected from among 6.30±0.2, 6.87±0.2, 8.58±0.2, 9.02±0.2, 10.26±0.2, 12.46±0.2, 14.71±0.2, 15.50±0.2, 17.42±0.2, 18.27±0.2, 19.56±0.2, 20.20±0.2, 21.55±0.2, 22.28±0.2 and 28.31±0.2.

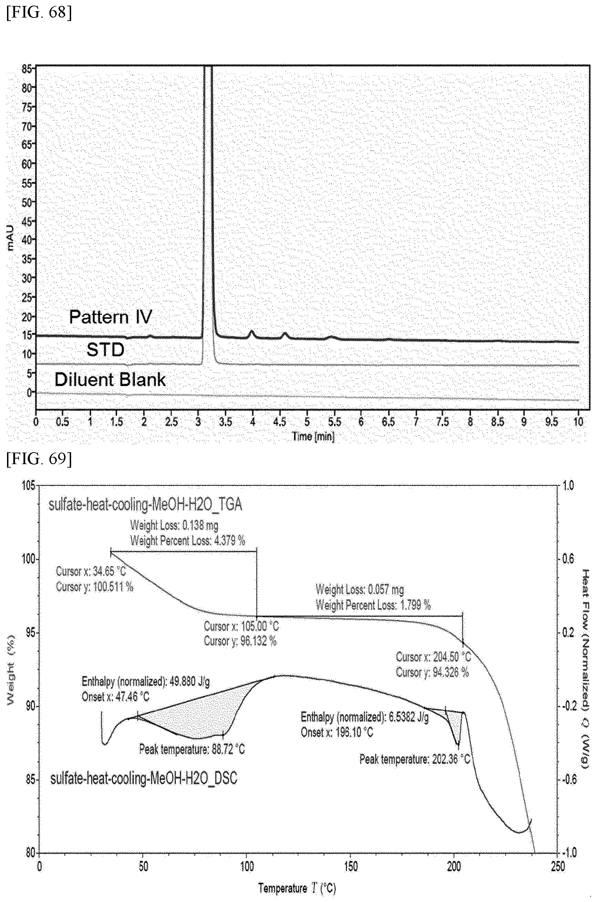

The XRPD result of FIG. 67 showed that the XRPD of Crystalline form IV was different from that of Crystalline form III, and the HPLC result of FIG. 68 showed 0.73% degradation with a purity of 99.27% (RT=3.151 min). The DSC graph of FIG. 69 showed TGA losses of 4.38% and 1.80% at about 90 °C and 200 °C, respectively. The result of DSC, in which heating was performed at 105 °C for 30 minutes, showed that water evaporated, which indicates that Crystalline form IV is a hydroxide, but when comparing the DSC result overlaid with a DSC/TGA overlay without water removal, the same pattern was shown. This indicates that Crystalline form IV of the sulfate could reversibly revert back to a hydroxide after being dried. Also, the results were consistent when comparing the XRPD result overlaid with a DSC/TGA overlay without water removal after vacuum drying at 50 °C for 5 days.

### Example 6-5: Identification of Crystalline form V

Crystalline form V was produced in the slurry method of Example 5-1 and the water activity of 0.501 of Example 5-8.

**[Table 19]**

| **List of characteristic XRPD peaks of Crystalline form V of sulfate** | | | |
|---|---|---|---|
| 2-Theta | d(A) | Counts | Intensity % |
| 6.876 | 12.845 | 2180 | 100.0 |
| 7.995 | 11.050 | 156 | 4.4 |
| 11.453 | 7.720 | 164 | 4.2 |
| 13.495 | 6.556 | 357 | 12.7 |
| 16.173 | 5.476 | 422 | 14.8 |
| 17.066 | 5.191 | 406 | 12.9 |
| 17.102 | 5.181 | 386 | 11.9 |
| 18.303 | 4.843 | 529 | 17.8 |
| 18.535 | 4.783 | 699 | 25.7 |
| 19.086 | 4.646 | 2260 | 99.4 |
| 19.707 | 4.501 | 758 | 28.4 |
| 20.151 | 4.403 | 1030 | 41.3 |
| 20.523 | 4.324 | 491 | 16.0 |
| 22.429 | 3.961 | 260 | 6.1 |
| 22.801 | 3.897 | 307 | 8.3 |
| 23.125 | 3.843 | 337 | 9.7 |
| 24.183 | 3.677 | 222 | 4.2 |
| 24.882 | 3.576 | 237 | 4.1 |
| 25.868 | 3.442 | 649 | 22.7 |
| 27.734 | 3.214 | 294 | 5.7 |
| 28.548 | 3.124 | 479 | 14.8 |
| 29.204 | 3.055 | 329 | 8.2 |
| 32.206 | 2.777 | 325 | 8.7 |

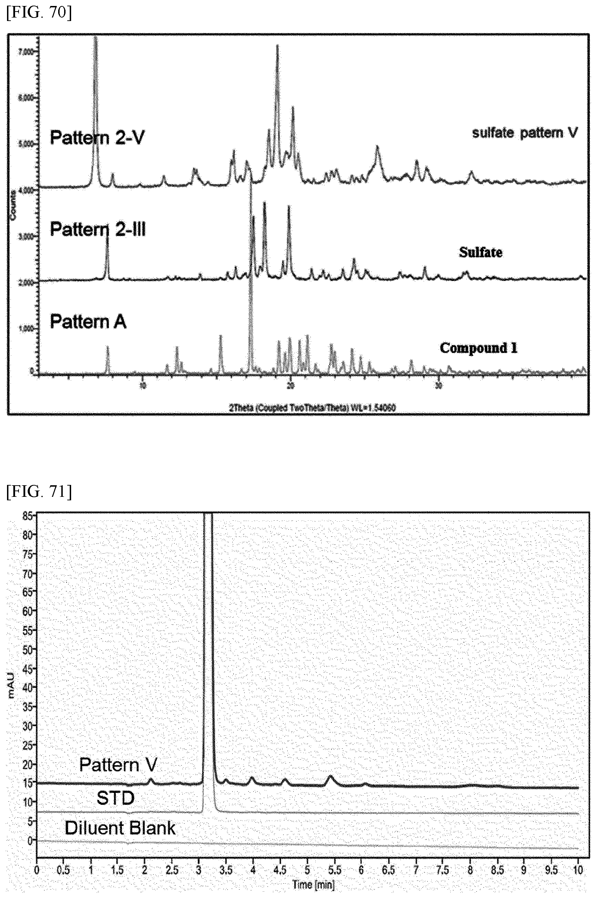

FIG. 70 and Table 19 show an XRPD result of Crystalline form V of the sulfate. Crystalline form V is identified with an X-ray powder diffraction pattern having at least 4 diffraction peaks at 2[θ] values selected from among 6.88±0.2, 13.50±0.2, 16.17±0.2, 17.07±0.2, 17.10±0.2, 18.30±0.2, 18.54±0.2, 19.09±0.2, 19.71±0.2, 20.15±0.2, 20.52±0.2, 25.87±0.2 and 28.55±0.2.

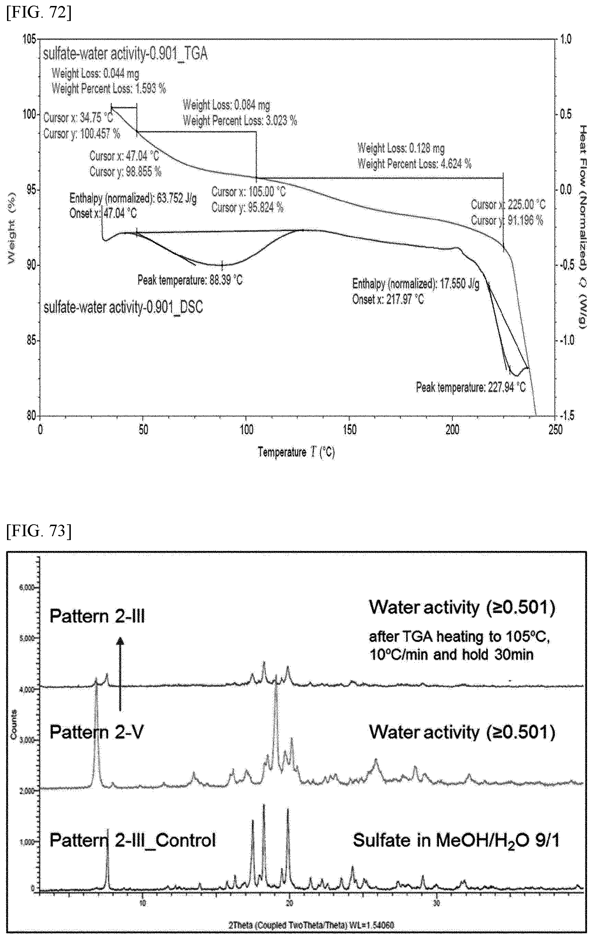

The XRPD result of FIG. 70 showed that the XRPD of Crystalline form V was different from that of Crystalline form III, and the HPLC result of FIG. 71 showed 1.86% degradation with a purity of 98.14% (RT=3.149 min). The DSC graph of FIG. 72 showed one TGA loss peak at 80 to 100 °C. As shown in FIG. 73, the XRPD of Crystalline form V after heating to 105 °C reverted back to Pattern 2-III, which indicates that Crystalline form V is a hydrate, not a novel pattern.

### Example 6-6: Identification of Crystalline form VI

Crystalline form VI was produced by a slurry method in DMF at 25 °C and 50 °C for 1 week.

**[Table 20]**

| **List of characteristic XRPD peaks of Crystalline form VI of sulfate** | | | |
|---|---|---|---|
| 2-Theta | d(A) | Counts | Intensity % |
| 6.514 | 13.558 | 1578 | 26.7 |
| 8.502 | 10.391 | 418 | 5.1 |
| 9.162 | 9.645 | 203 | 1.1 |
| 12.043 | 7.343 | 231 | 0.9 |
| 12.534 | 7.057 | 422 | 4.3 |
| 15.728 | 5.630 | 1241 | 17.1 |
| 16.474 | 5.377 | 643 | 4.9 |
| 17.030 | 5.202 | 5711 | 97.9 |
| 17.407 | 5.090 | 916 | 8.8 |
| 18.013 | 4.921 | 623 | 2.7 |
| 18.299 | 4.844 | 823 | 6.1 |
| 19.006 | 4.666 | 1548 | 19.0 |
| 19.851 | 4.469 | 5959 | 100.0 |
| 20.453 | 4.339 | 2000 | 26.6 |
| 20.817 | 4.264 | 950 | 7.1 |
| 21.530 | 4.124 | 1013 | 8.2 |
| 22.654 | 3.922 | 745 | 3.4 |
| 24.200 | 3.675 | 1816 | 24.1 |
| 24.650 | 3.609 | 725 | 4.3 |
| 25.398 | 3.504 | 864 | 7.6 |
| 26.107 | 3.410 | 795 | 6.9 |
| 28.473 | 3.132 | 713 | 6.1 |
| 28.703 | 3.108 | 551 | 3.2 |
| 29.151 | 3.061 | 586 | 4.1 |
| 29.427 | 3.033 | 510 | 2.8 |
| 31.987 | 2.796 | 488 | 3.1 |
| 34.070 | 2.629 | 358 | 1.1 |
| 35.520 | 2.525 | 408 | 2.3 |
| 37.002 | 2.428 | 320 | 0.8 |
| 39.492 | 2.280 | 306 | 0.8 |

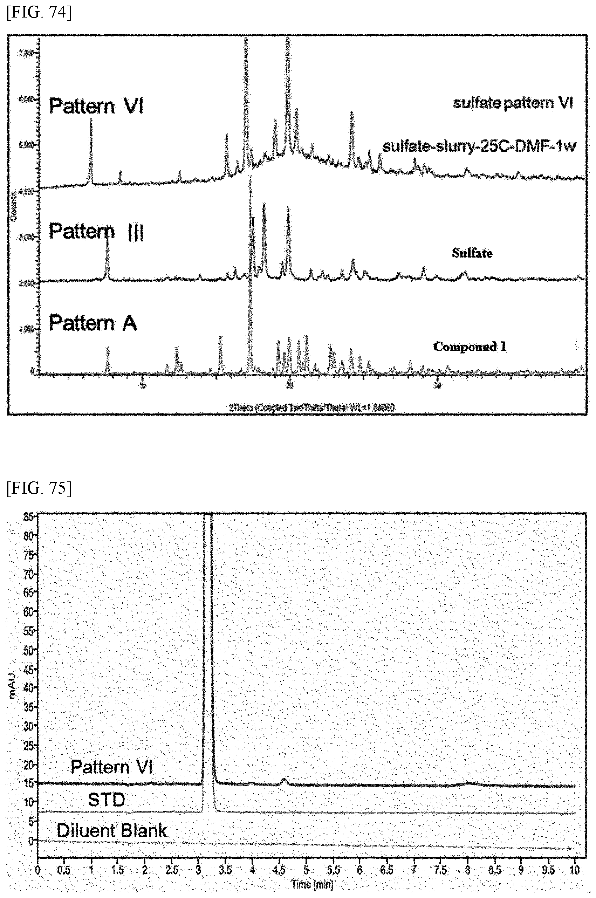

FIG. 74 and Table 20 show an XRPD result of Crystalline form VI of the sulfate. The Crystalline form VI is identified with an X-ray powder diffraction pattern having at least 4 diffraction peaks at 2[θ] values selected from among 6.51±0.2, 15.73±0.2, 17.03±0.2, 19.01±0.2, 19.85±0.2, 20.45±0.2 and 24.20±0.2.

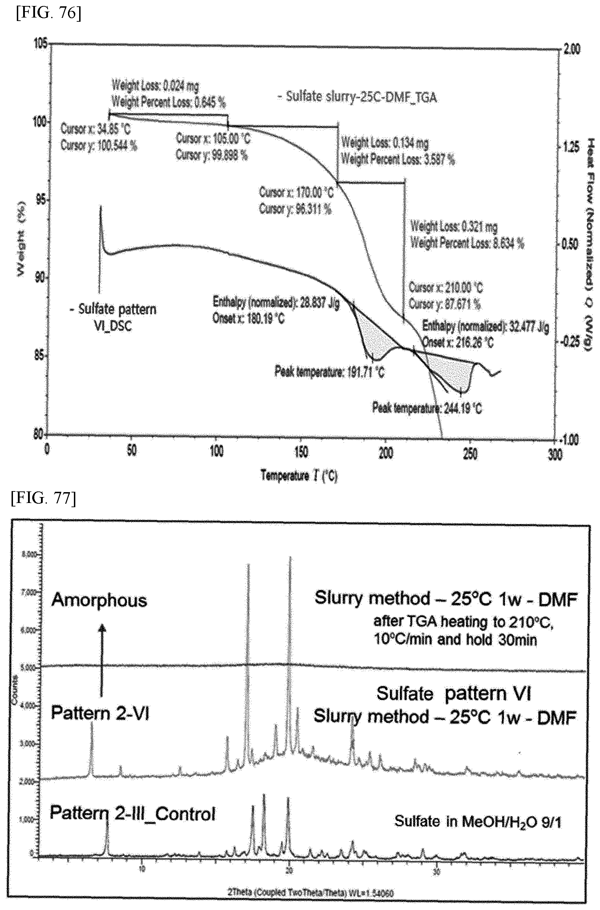

The XRPD result of FIG. 74 showed that the XRPD of Crystalline form VI was different from that of Crystalline form III, which indicates that pattern VI may be a novel pattern. The HPLC result of FIG. 75 showed 0.78% degradation with a purity of 99.22% (RT=3.149 min). The DSC graph of FIG. 76 showed a TGA loss of 12.87% until 210 °C, and the XRPD of Crystalline form VI was changed to an amorphous state after heating to 210 °C (FIG. 77). It is likely a DMF solvate.

### Example 6-7: Identification of Crystalline form VII

Crystalline form VII was produced by the slurry method in acetone/water (1:1) at 50 °C for 4 weeks.

**[Table 21]**

| **List of characteristic XRPD peaks of Crystalline form VII of sulfate** | | | |
|---|---|---|---|
| 2-Theta | d(A) | Counts | Intensity % |
| 8.153 | 10.835 | 460 | 12.6 |
| 16.304 | 5.432 | 3224 | 100.0 |
| 16.611 | 5.333 | 1241 | 36.0 |
| 24.541 | 3.624 | 385 | 9.0 |
| 24.993 | 3.560 | 150 | 1.6 |
| 32.895 | 2.721 | 317 | 7.9 |
| 33.566 | 2.668 | 164 | 2.9 |

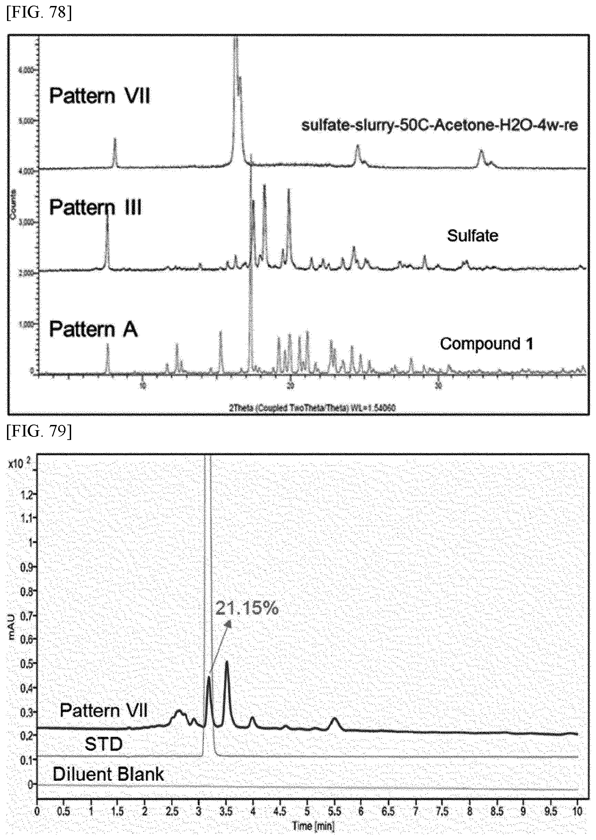

FIG. 78 and Table 21 show an XRPD result of Crystalline form VII of the sulfate. Crystalline form VII is identified with an X-ray powder diffraction pattern having at least 3 diffraction peaks at 2[θ] values selected from among 8.15±0.2, 16.30±0.2, 16.61±0.2 and 24.54±0.2.

The XRPD result of FIG. 78 showed that the XRPD of Crystalline form VII was different from that of Crystalline form III, and the HPLC result of FIG. 79 showed excessive degradation with a purity of only 21.15%, and since a degradation rate is the main factor responsible for the difference in XPRD, Crystalline form VII was a degradation product.

### Example 6-8: Identification of Crystalline form VIII

A novel crystalline form was found by heating Crystalline form IV to remove water and maintaining the same for 10 minutes and named Crystalline form VIII.

**[Table 22]**

| **List of characteristic XRPD peaks of Crystalline form VIII of sulfate** | | | |
|---|---|---|---|
| 2-Theta | d(A) | Counts | Intensity % |
| 6.350 | 13.909 | 2002 | 100.0 |
| 6.775 | 13.036 | 993 | 40.7 |
| 8.012 | 11.027 | 422 | 7.6 |
| 8.356 | 10.573 | 801 | 30.0 |
| 9.396 | 9.405 | 579 | 16.4 |
| 10.427 | 8.477 | 477 | 8.3 |
| 12.274 | 7.205 | 532 | 6.8 |
| 12.498 | 7.077 | 578 | 9.0 |
| 13.983 | 6.328 | 736 | 15.5 |
| 15.975 | 5.544 | 844 | 16.0 |
| 17.846 | 4.966 | 764 | 6.7 |
| 19.033 | 4.659 | 834 | 9.0 |
| 19.660 | 4.512 | 845 | 8.9 |
| 20.427 | 4.344 | 911 | 12.4 |
| 21.149 | 4.197 | 1189 | 28.7 |
| 21.401 | 4.149 | 1315 | 36.2 |
| 21.614 | 4.108 | 1425 | 42.7 |

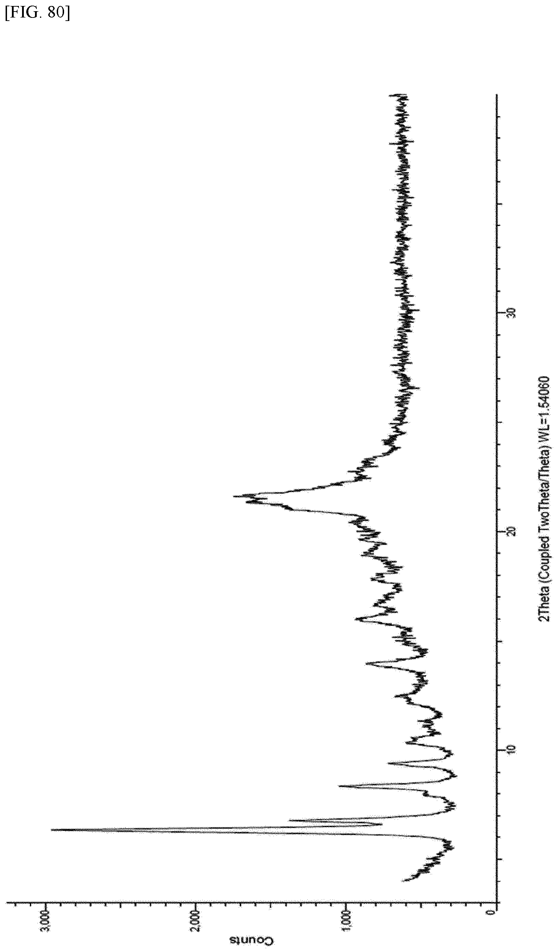

FIG. 80 and Table 22 show an XRPD result of Crystalline form VIII of the sulfate. Crystalline form VIII is identified with an X-ray powder diffraction pattern having at least 4 diffraction peaks at 2[θ] values selected from among 6.35±0.2, 6.78±0.2, 8.36±0.2, 9.40±0.2, 13.98±0.2, 15.98±0.2, 20.43±0.2, 21.15±0.2, 21.40±0.2, and 21.61±0.2.

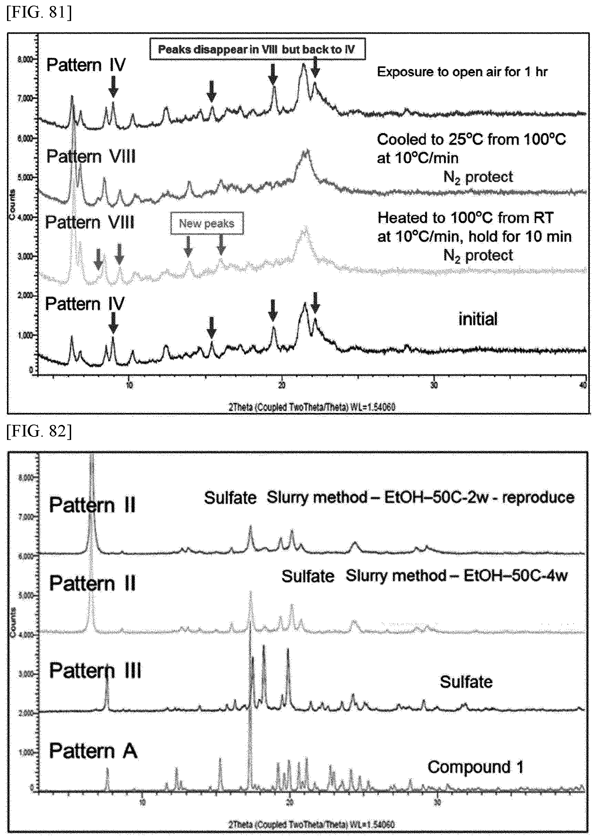

Crystalline form VIII was shown to be different from Crystalline form IV and maintained upon nitrogen filling protection after cooling to 25 °C. However, considering that Crystalline form VIII disappeared within 1 hour when exposed to air, metastable Crystalline form VIII was shown to be a novel crystalline form that absorbs water and is easily changed to Crystalline form IV as a new pattern (FIG. 81).

### Example 7: Scale-up of crystalline form

4 Crystalline forms II, III, IV and VI of the compound of Chemical Formula 1 were subjected to scale-up. As a result, the scaled-up crystalline form could be prepared to be the same as the existing crystalline form.

### Example 7-1: 200 mg scale-up of Crystalline form II of sulfate of compound of Chemical Formula 1

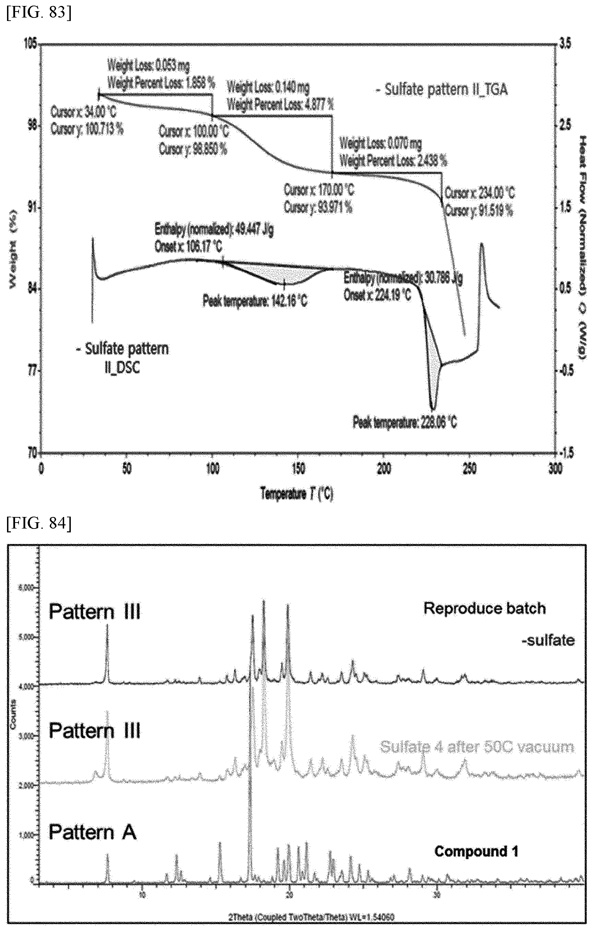

About 200 mg of the sulfate of the compound of Chemical Formula 1 was input into a 2 mL vial, 4 mL of ethanol was added, stirring was performed at 50 °C and 650 rpm for 2 weeks, vacuum drying was performed at 30 °C for 16 hours, and XRPD and TGA/DSC tests were conducted. According to the XRPD pattern and the DSC/TGA result, scaled-up Crystalline form II was the same as existing Crystalline form II. FIG. 82 shows an XRPD result of prepared Crystalline form II of the sulfate of the compound of Chemical Formula 1, and FIG. 83 shows a TGA/DSC result thereof.

### Example 7-2: 10 g scale-up of Crystalline form III of sulfate of compound of Chemical Formula 1

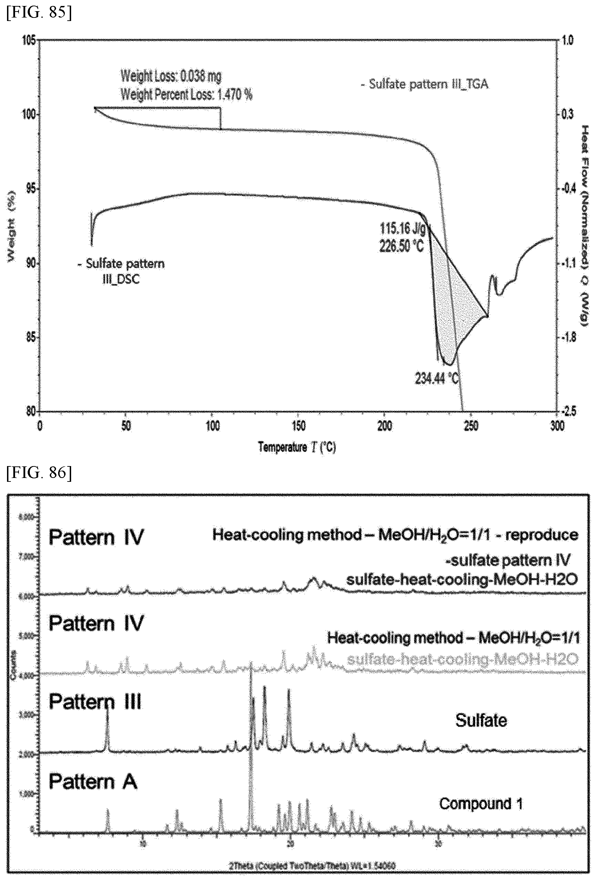

About 10 g of the free form of the compound of Chemical Formula 1 was dissolved in 149.333 mL of MeOH/H₂O (9/1) and stirred at 50 °C and 250 rpm for 15 minutes, and 1.1 equivalents (50.114 mL, 0.5 mol/L) of a solution containing sulfuric acid and MeOH/H₂O (9/1) was added dropwise for 8 hours. Afterward, the resulting mixture was maintained at 50 °C for 24 hours, cooled to 5 °C for 9 hours, and maintained at 5 °C for 24 hours. The suspension was filtered, washed with a MeO/H₂O (9/1) solution, and vacuum-dried at 50 °C for 3 days. 11.11 g of white powder was obtained with a yield of 90.75%. According to the XRPD pattern and the DSC/TGA result, scaled-up Crystalline form III was the same as existing Crystalline form III. FIG. 84 shows an XRPD result of prepared Crystalline form III of the compound of Chemical Formula 1, and FIG. 85 shows a TGA/DSC result thereof.

### Example 7-3: 500 mg scale-up of Crystalline form IV of sulfate of compound of Chemical Formula 1

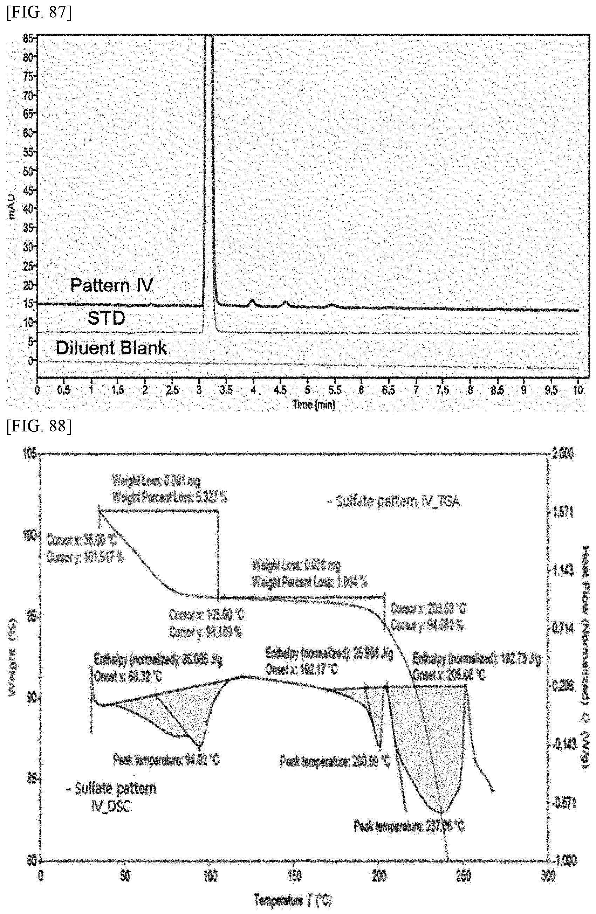

About 500 mg of the sulfate of the compound of Chemical Formula 1 was input into a 100 mL vial, and 50 mL of a MeOH/H₂O (1:1) solution was added and completely dissolved at 50 °C while slowly heating at 650 rpm. The temperature was lowered from 50 °C to 0 °C at 0.5 °C /min and then maintained at 0 °C for 1 day. The precipitate was filtered and vacuum-dried at 30 °C for 16 hours. According to the XRPD pattern, the HPLC spectrum and the DSC/TGA result, scaled-up Crystalline form IV was the same as existing Crystalline form IV (FIGS. 86 to 88).

### Example 7-4: 300 mg scale-up of Crystalline form VI of sulfate of compound of Chemical Formula 1

About 300 mg of the sulfate of the compound of Chemical Formula 1 was input into a 2 mL vial, 0.3 mL of DMF was added, stirring was performed at 25 °C and 650 rpm for 2 days, and centrifugation was performed to obtain a solid. According to the XRPD pattern and the DSC/TGA result, scaled-up Crystalline form VI was the same as existing Crystalline form VI.

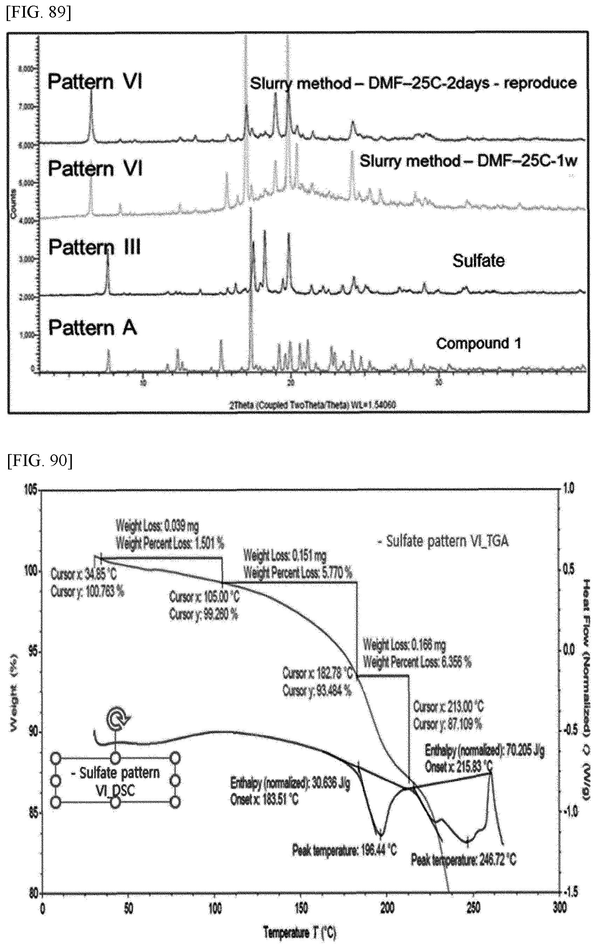

FIG. 89 shows an XRPD result of prepared Crystalline form VI of the sulfate of the compound of Chemical Formula 1, and FIG. 90 shows a TGA/DSC result thereof.

### Example 8: Stability test of Crystalline form III of sulfate of compound of Chemical Formula 1

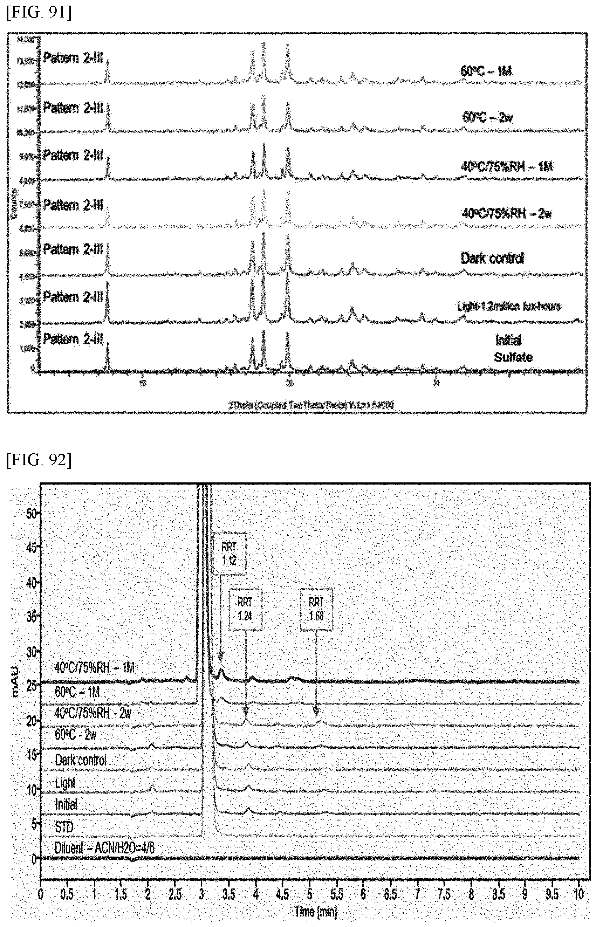

The chemical and physical stability of Crystalline form III were studied under conditions of light, light-shielding, 60 °C and 40 °C/75% RH for 1 month. About 1 mg of Crystalline form III of the sulfate of the compound of Chemical Formula 1 was input into a 2 mL glass bottle, covered with aluminum foil with several holes, and stored in a stability test chamber at 60 °C and 40 °C/75% RH. In the light stability test, the crystalline form was stored in a light stability test chamber without a lid and thus exposed to a total illuminance of 1.2 million lux-hr, and a sample vial completely covered with an aluminum foil was regarded as a light-shielded control (Dark control). Appearance by visual observation for 2 weeks, 1 month, 2 months and 3 months was recorded, and purity assessment and XPRD data collection for residual solid contents were performed. The results thereof are shown in Tables 23 and 24, FIG. 91 shows an XRPD change during the stability test of Crystalline form III of the sulfate of the compound of Chemical Formula 1, and FIG. 92 shows an HPLC change.

**[Table 23]**

| **Stability test of Crystalline form III of sulfate of compound of Chemical Formula 1** | | | | | | | |
|---|---|---|---|---|---|---|---|
| Starting materials | Conditions | | Appearance | XRPD patterns | Final purity | Purity change | Assay |
| Pattern III (Anhydrate, non-solvate) | Initial | | White powder | Pattern III | 99.38% | | |
| | Light | | Taupe powder | Pattern III | 99.38% | -0.00% | 101.77% |
| | Dark | | White powder | Pattern III | 99.33% | -0.05% | 103.17% |
| | 60 °C | 2w | White powder | Pattern III | 99.24% | -0.14% | 101.08% |
| | | 1M | White powder | Pattern III | 99.25% | -0.13% | 106.79% |
| | | 2M | pending | pending | pending | pending | pending |
| | | 3M | pending | pending | pending | pending | pending |
| | 40 °C /75%RH | 2w | White powder | Pattern III | 98.77% | -0.61% | 99.42% |
| | | 1M | White powder | Pattern III | 98.71% | -0.67% | 102.84% |
| | | 2M | pending | pending | pending | pending | pending |
| | | 3M | pending | pending | pending | pending | pending |

**[Table 24]**

| **Stability test of Crystalline form III of sulfate of compound of Chemical Formula 1 and purity change** | | | | | | | | | |
|---|---|---|---|---|---|---|---|---|---|
| Peak # | RT (min) | RRT | Area of Sulfate (%) | | | | | | |
| | | | Initial | 1.2 million lux-hr | Dark control | 60 °C | | 40 °C/75%RH | |
| | | | | | | 2W | 1M | 2W | 1M |
| **1** | 1.512 | 0.50 | - | - | - | - | - | 0.069 | - |
| **2** | 1.888 | 0.63 | - | - | - | - | 0.168 | - | 0.183 |
| **3** | 2.022 | 0.67 | 0.204 | 0.226 | 0.209 | 0.231 | - | 0.226 | - |
| **4** | 2.036 | 0.68 | - | - | - | - | 0.056 | - | 0.061 |
| **5** | 2.481 | 0.82 | - | - | - | 0.052 | - | - | - |
| **6** | 2.704 | 0.91 | - | - | - | - | - | - | 0.102 |
| **7** | 3.021 | 1.00 | 99.319 | 99.382 | 99.326 | 99.237 | 99.247 | 98.769 | 98.711 |
| **8** | 3.362 | 1.11 | 0.069 | - | 0.069 | 0.114 | - | 0.206 | - |
| **9** | 3.355 | 1.12 | - | - | - | - | 0.325 | - | 0.518 |
| **10** | 3.75 | 1.24 | 0.215 | 0.221 | 0.219 | 0.211 | - | 0.321 | - |
| **11** | 3.938 | 1.32 | - | - | - | - | 0.088 | - | 0.149 |
| **12** | 4.299 | 1.42 | 0.08 | 0.073 | 0.078 | 0.054 | - | 0.084 | - |

As a result, Crystalline form III was stable under light and light-shielding conditions and stable at 60 °C for 1 month, and the purity thereof was decreased by only 0.67% at 40 °C for 1 month.

## Claims

1. A sulfate of a compound of the following Chemical Formula 1.

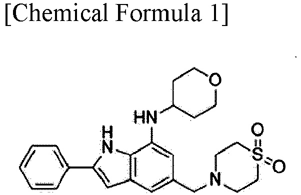

2. A crystalline form I of the sulfate of the compound of Chemical Formula 1 according to Claim 1, wherein the crystalline form is identified with an X-ray powder diffraction pattern having at least 4 diffraction peaks at 2[θ] values selected from among 6.63±0.2, 17.34±0.2, 17.76±0.2, 18.70±0.2, 18.93±0.2, 20.24±0.2, 20.83±0.2, 21.56±0.2, 24.66±0.2 and 29.00±0.2.

3. A crystalline form II of the sulfate of the compound of Chemical Formula 1 according to Claim 1, wherein the crystalline form is identified with an X-ray powder diffraction pattern having at least 4 diffraction peaks at 2[θ] values selected from among 6.57±0.2, 13.91±0.2, 16.05±0.2, 17.36±0.2, 18.41±0.2, 19.16±0.2, 19.36±0.2, 20.11±0.2, 20.77±0.2 and 24.31±0.2.

4. A crystalline form III of the sulfate of the compound of Chemical Formula 1 according to Claim 1, wherein the crystalline form is identified with an X-ray powder diffraction pattern having at least 4 diffraction peaks at 2[θ] values selected from among 7.66±0.2, 16.32±0.2, 17.53±0.2, 18.05±0.2, 18.28±0.2, 18.95±0.2, 19.49±0.2, 19.91±0.2, 21.44±0.2, 22.24±0.2, 23.52±0.2, 24.29±0.2, 25.10±0.2, 27.37±0.2, 29.07±0.2 and 31.90±0.2.

5. A crystalline form IV of the sulfate of the compound of Chemical Formula 1 according to Claim 1, wherein the crystalline form is identified with an X-ray powder diffraction pattern having at least 4 diffraction peaks at 2[θ] values selected from among 6.30±0.2, 6.87±0.2, 8.58±0.2, 9.02±0.2, 10.26±0.2, 12.46±0.2, 14.71±0.2, 15.50±0.2, 17.42±0.2, 18.27±0.2, 19.56±0.2, 20.20±0.2, 21.55±0.2, 22.28±0.2 and 28.31±0.2.

6. A crystalline form V of the sulfate of the compound of Chemical Formula 1 according to Claim 1, wherein the crystalline form is identified with an X-ray powder diffraction pattern having at least 4 diffraction peaks at 2[θ] values selected from among 6.88±0.2, 13.50±0.2, 16.17±0.2, 17.07±0.2, 17.10±0.2, 18.30±0.2, 18.54±0.2, 19.09±0.2, 19.71±0.2, 20.15±0.2, 20.52±0.2, 25.87±0.2 and 28.55±0.2.

7. A crystalline form VI of the sulfate of the compound of Chemical Formula 1 according to Claim 1, wherein the crystalline form is identified with an X-ray powder diffraction pattern having at least 4 diffraction peaks at 2[θ] values selected from among 6.51±0.2, 15.73±0.2, 17.03±0.2, 19.01±0.2, 19.85±0.2, 20.45±0.2 and 24.20±0.2.

8. A crystalline form VII of the sulfate of the compound of Chemical Formula 1 according to Claim 1, wherein the crystalline form is identified with an X-ray powder diffraction pattern having at least 3 diffraction peaks at 2[θ] values selected from among 8.15±0.2, 16.30±0.2, 16.61±0.2 and 24.54±0.2.

9. A crystalline form VIII of the sulfate of the compound of Chemical Formula 1 according to Claim 1, wherein the crystalline form is identified with an X-ray powder diffraction pattern having at least 4 diffraction peaks at 2[θ] values selected from among 6.35±0.2, 6.78±0.2, 8.36±0.2, 9.40±0.2, 13.98±0.2, 15.98±0.2, 20.43±0.2, 21.15±0.2, 21.40±0.2, and 21.61±0.2.

10. A method for preparing the sulfate of the compound of Chemical Formula 1 according to Claim 1, the method comprising:
stirring a solution including a compound of the following Chemical Formula 1 and a solvent selected from ethanol, methyl ethyl ketone (MEK), ethyl acetate, methanol and acetone under heating; and
drop-wise adding, to the solution, a solution including the same solvent as the solvent and sulfuric acid and performing cooling.

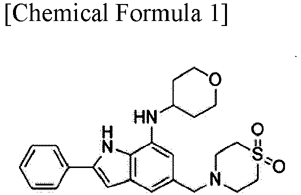

11. A method for preparing the crystalline form III of the sulfate of the compound of Chemical Formula 1 according to Claim 4, the method comprising:
stirring a solution including a compound of the following Chemical Formula 1 and methanol under heating; and
drop-wise adding, to the solution, a solution including methanol and sulfuric acid and performing cooling.

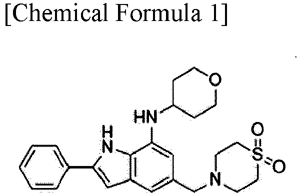

12. A pharmaceutical composition comprising the sulfate of the compound of Chemical Formula 1 according to Claim 1 or the crystalline form of the sulfate of the compound of Chemical Formula 1 according to any one of Claim 2 to Claim 9, and a pharmaceutically acceptable carrier.

13. A pharmaceutical composition comprising the sulfate of the compound of Chemical Formula 1 according to Claim 1 or the crystalline form of the sulfate of the compound of Chemical Formula 1 according to any one of Claim 2 to Claim 9, and a pharmaceutically acceptable carrier, for preventing or treating necrosis and a necrosis-related disease selected from the following:
acute/chronic liver disease, dementia, Parkinson's disease, Huntington's disease, ischemic disease, diabetes, pancreatitis, bacterial/viral sepsis, necrotizing proctitis, cystic fibrosis, rheumatoid arthritis, degenerative arthritis, nephropathy, bacterial infection, viral infection, multiple sclerosis, leukemia, lymphoma, neonatal respiratory distress syndrome, asphyxia, tuberculosis, endometriosis, angiasthenia, psoriasis, chilblain, steroid treatment complications, gangrene, pressure sores, hemoglobinuria, burn, hyperthermia, Crohn's disease, celiac disease, compartment syndrome, spinal cord injury, glomerulonephritis, muscular dystrophy, mycoplasma disease, anthrax, Andersen's disease, congenital mitochondrial disease, phenylketonuria, placental infarction, syphilis, aseptic necrosis; in addition, necrosis and associated diseases caused by drugs and toxic substances are selected from the group consisting of the necrosis associated with alcoholism, the exposure to, and/or administration and/or self-administration of, cocaine, antibiotics, anti-cancer drugs, nonsteroidal anti-inflammatory drugs (NSAIDSs), cyclosporine, chemical toxins, poison gas, agrochemicals, heavy metals, or injury due to the exposure to radiation/UV and associated necrosis thereof, acute/chronic kidney disease, traumatic brain injury, amyotrophic lateral sclerosis, necrotizing colitis, viral infection, skin disease including psoriasis and allergic dermatitis, organ preservation / organ transplant, acute lung injury syndrome / acute pulmonary disease, pneumonia, tuberculosis, asthma, pulmonary hypertension, inflammatory pulmonary disease including chronic obstructive pulmonary disease, idiopathic pulmonary fibrosis and cystic fibrosis, demyelinating disease including demyelination and amyotrophic lateral sclerosis (ALS), hypertension including pulmonary hypertension, stroke, prion disease, epilepsy, ataxia, migraines, reduced cognitive ability, seizure, tremors, mental disorders, insulin resistance, hyperlipidemia, atherosclerosis, inflammatory bowel disease (IBD) including Crohn's Disease and ulcerative colitis, various cancers and metastasis of cancer, visual impairment-associated diseases such as retinitis pigmentosae, optic neuropathy, cataracts, glaucoma, anemia, cholestasis, hypoparathyroidism, pancytopenia, pancreatic disorder, lactic acidosis, lactacidemia, loss of hearing, short stature, ileus, cardiac conduction defect, cardiomyopathy, endometriosis, infertility, early menopause, muscular atrophy diseases including limb gridle / Becker muscular dystrophy (LGMD/BMD) and Duchenne muscular dystrophy (DMD), aging and aging-related illnesses, and mucositis.

14. A composition comprising the sulfate of the compound of Chemical Formula 1 according to Claim 1 or the crystalline form of the sulfate of the compound of Chemical Formula 1 according to any one of Claim 2 to Claim 9, for inducing differentiation of stem cells into cardiomyocytes.
